# EUROPEAN PATENT SPECIFICATION

(11) **EP 2 102 654 B1**
(45) Date of publication and mention of the grant of the patent: **13.08.2014**
(21) Application number: 08700137.6
(22) Date of filing: 07.01.2008
(51) Int. Cl.: G01N 33/50

(54) **METHOD AND KIT FOR DETECTING HERBICIDE RESISTANCE IN PLANTS**
VERFAHREN UND KIT ZUM NACHWEIS HERBIZIDRESISTENZ IN PFLANZEN
METHODE ET KIT DE DETECTION RESISTANCE AUX HERBICIDES CHEZ PLANTES

(30) Priority: 07.01.2007 DK 200700024
(43) Date of publication of application: 23.09.2009
(73) Proprietor: Aarhus Universitet, 8000 Aarhus C (DK)
(72) Inventor: RAVN, Helle Weber, DK-8600 Silkeborg (DK); KUDSK, Per Nielsen, DK-4200 Slagelse (DK); MATHIASSEN, Solvejg K., DK-4700 Næstved (DK)
(74) Representative: Høiberg A/S
(86) International application number: PCT/DK2008/050004
(87) International publication number: WO 2008/080410

(56) References cited:
- WO-A-01/86303
- WO-A-01/92879
- WO-A-03/025223
- WO-A-03/031937
- US-A- 6 060 039

## Description

### Field of invention

The present invention relates to a method and a kit for testing the presence of resistance in living organisms. Especially the invention relates to a method and a kit for testing the presence of resistance in plants at an early time. More specifically the invention relates to a simple and fast method to detect resistance using biochemical compounds in living organisms which has developed resistance to herbicides before and/or after exposure to chemical stress such as pesticides (herbicides). The kit and method correlate the type of biochemical compounds e.g. phytochemical compounds in plants and/or the level of the biochemical compounds in living organism exposed to pesticides with the presence of biochemical compounds related to resistance. The kit provides a simple and fast method to detect the existence of resistance, e.g. to detect herbicide resistance in weed plant or predict the time a plant develop resistance e.g. the time a weed plant develop resistance to a herbicide. It is also possible to detect crop plants which are tolerant to herbicide and which may be present in fields cultivated with another crop plant.

### Background of invention

Plants, animals and other living organisms including fungi are exposed to stress continuously or temporarily throughout their life time. It is known that different types of stress, different exposure times and different amounts of a single stress type can influence differently depending on the species of e.g. plants or animals. If the stress is due to chemical compounds such as pesticides some of the individual organism may develop resistance to this or these pesticides.

When one or a few individuals of a population develop resistance in respect of a pesticide, these individuals are favoured in their growth in environments where the pesticide treatments are performed frequently. With time the population of living organism will develop to include more individuals being resistant to the pesticide treatments.

Resistance in a living organism, especially in organism being damaging or harmful to e.g. crop plants and hereby reducing the yield of the crops can have far-reaching consequences and it is thus an advantage if the development of resistance can be detected at an early stage, this being well before a pesticide treatment has lost the effect in a greater part of the population of the living organism.

Resistance to a pesticide means that the organism is no longer sensitive to the pesticide either due to a mutation resulting in an insensible target site (also referred to as "target site resistance") or the organism has become capable of metabolising the pesticide resulting in a reduced and short-lived effect of the pesticide (also referred to as "metabolic resistance"). In this context "target site resistance" need not be due to only a single mutation within the organism, but may be caused by two or more mutations in the genome of the organism.

World-wide herbicide-resistance is an increasing problem. Since 1960 the number of unique cases have increased to more than 300.

Within the last 10 years particularly the number of cases of resistance to ALS and ACC-ase inhibitors has increased and most recently, resistance to glyphosate has been reported.

WO 03/031937 describes the identification of polymorphic markers of herbicide resistance in plants by isolation genomic DNA from a herbicide susceptible plant and a herbicide resistant plant of the same species and performing genetic analysis on the DNA to identify the polymorphic markers of the herbicide resistance. The polymorphic markers are for identifying herbicide resistant and herbicide susceptible weeds.

WO 01/86303 describes detecting the polypeptide glutathionene S-transferase in a weed. The polypeptide is connected with resistance of the weed to herbicides. The polypeptide is detected by an antiserum which binds with the polypeptide. The bound antiserum can be visualised e.g. by ELISA.

In the present invention the differences in the chemical composition of living organism and especially of phytochemical composition of weed plants is used to develop a new simple method to detect resistance in weed plants and other living organism. The method and kit can be used by farmers or advisors to determine whether insatisfactory weed control in a field is due to development of herbicide resistance in the weed species. The farmer will be able to adjust the weed control method in relation to the test result.

Phytochemical compounds have been used as biomarkers to obtain a biomarker pattern in plants exposed to stress (WO 01/92879). A biomarker pattern in plants is defined as the changes in the composition and content of phytochemical compounds detected in plants after exposure to stress such as herbicides.

To be able to prevent or delay resistance development in living organism it is important to identify the resistance at an early stage. An early detection of the existence of resistance in e.g. weed, pest and/or pathogenic fungi is of special interest to the farming industry, mainly to reduce the risk of using pesticides not having an effect any more. An early detection of resistance is also of interest in other fields such as in the control of insects living on or close to humans or influencing the health of humans e.g. in respect of lice, mosquitoes and rats.

The present invention discloses a simple, fast and highly sensitive method for testing the existence of resistance in living organism towards chemicals such as in plants short time after exposure to chemicals such as pesticides. The method takes advantage of a change in the composition of chemical compounds when living organism are exposed to stress, and these compounds can be used as biomarkers in the material from a living organism when exposed to stress. Particularly, the present invention relates to a method of testing for the presence of resistance in weed plants exposed to chemical treatment with herbicides.

### Summary of invention

In a main aspect the invention relates to a method as claimed in claim 1.

Another aspect of the invention relates to a method of providing a standard visualising scale for material from a specific living plant that has or has not been exposed to a specific herbicide as claimed in claim 10.

In an aspect the invention relates to an assay kit for testing for resistance in a living plant as claimed in claim 13.

### Description of drawings

Fig. 1. The development of resistance factors in different biotypes of the plant *Stellaria media* exposed to herbicide treatments including the active ingredients iodosulfuron, tribenuron and florasulam.
Fig. 2. In an experiment with *Papaver rhoeas*/*Hussar OD,* a mixture of I₁, A₁ AD_{α1} (1:1:1) 4 drops + 10 drops of plant extract (50 mg/ml extracted with water) + 15 drops of conc. sulphuric acid present the colour picture of Fig. 2. The colours are (from left to right) PANTONE^{®} no. 535 (blue-gray); no. 452 (green-gray); no. 7544 (gray), no. 415 (gray-green).
Fig. 3. Thin Layer Chromatography (TLC) of Extract from *Papaver rhoeas* unexposed and exposed to Hussar. TLC 1): Plate: Silica gel 60; Solvent: Toluene: Ethyl acetate: Diethylamine (70:20:10). Further information in the Examples.
Fig. 4. Thin Layer Chromatography (TLC) of Extract from *Papaver rhoeas* unexposed and exposed to Hussar. TLC 2): Plate: Cellulose; Solvent: 15% Acetic acid in water. Further information in the Examples.
Fig. 5. Thin Layer Chromatography (TLC) of Extract from *Papaver rhoeas* unexposed and exposed to Hussar. TLC 3) Plate: Silica gel 60; Solvent: n-butanol : Methanol : Water (30:30:40). Further information in the Examples.
Fig. 6. Thin Layer Chromatography (TLC) of Extract from *Papaver rhoeas* unexposed and exposed to Hussar. TLC 4) Plate: Cellulose; Solvent: Isopropanol : Acetic acid : Water (70:5:25). Further information in the Examples.
Fig. 7. Thin Layer Chromatography (TLC) of Extract from *Papaver rhoeas* unexposed and exposed to Hussar. TLC 5) Plate: Cellulose; Solvent: 2% Acetic acid in Water. Further information in the Examples.
Fig. 8. Thin Layer Chromatography (TLC) analysis of *Stellaria media* unexposed or exposed to Hussar. (TLC 1): Plate: Cellulose; Solvent: S1; Aq & Et, T = luteoline.
Further information in the Examples.
Fig. 9. Thin Layer Chromatography (TLC) analysis of *Stellaria media* unexposed or exposed to Hussar. TLC 2): Plate: Cellulose; Solvent: S2. Further information in the Examples.
Fig. 10. Thin Layer Chromatography (TLC) analysis of *Stellaria media* unexposed or exposed to Hussar. TLC 3) Plate: Cellulose; Solvent S3. Aq & Et. Further information in the Examples.
Fig. 11. Thin Layer Chromatography (TLC) analysis of *Stellaria media* unexposed or exposed to Hussar. TLC 4) Plate: Silica; Solvent: S4; Aq & Et. Further information in the Examples.
Fig. 12. Thin Layer Chromatography (TLC) analysis of *Stellaria media* unexposed or exposed to Hussar. TLC 5) Plate: Silica; Solvent S5; Aq. T = glucose & rhamnose (blue). Further information in the Examples.
Fig. 13. Thin Layer Chromatography (TLC) analysis of *Stellaria media* unexposed or exposed to Hussar. TLC 6) Plate: Cellulose; Solvent S6; Aq. T = apigenine. Further information in the Examples.

### Detailed description of the invention

By the present invention it has become possible to detect whether a living organism has developed resistance e.g. to a chemical compound such as a pesticide by applying a simple and highly sensitive method of testing. Living organism including plants may produce different amounts and/or different types of chemical compounds following exposure to stress effects when compared to the living organism in a no-stress environment. These chemical compounds in living organism not exposed to stress and in living organism exposed to stress can be used as biomarkers to determine the presence of resistance.

Especially it has become possible to detect resistance to pesticides in plants, pests and fungi before application of the pesticides or following application of a pesticide when the effect of a pesticide treatment has no or only little effect. The invention relates to a simple and fast field method to detect biochemical compounds in living organism or phytochemical compounds in plants exposed to chemical stress such as pesticide treatment. The level and/or type of chemical/phytochemical compounds of the living organism can be correlated with development of biochemical compounds being detected as resistance factors in the living organism.

An aspect of the invention relates to a method for detecting resistance in a living organism, said method comprises
- obtaining at least one living organism, which has been exposed to a pesticide or exposing said at least one living organism to a pesticide,
- after a period of time determined from the time of the pesticide exposure, selecting at least a part of one of the living organism which is not dead due to the exposure to said pesticide,
- providing an assayable form of said part of living organism, said assayable form of living organism material comprising at least one group of chemical compounds,
- visualising said at least one group of chemical compounds optionally by a visual and/or UV-light detection,
- correlating said visualising of at least one group of biochemical compounds to a standard visualising scale of said at least one group of chemical compounds,
- assessing whether said living organism has developed resistance towards said pesticide.

An aspect of the invention relates to a method for detecting resistance in a living organism without exposure of herbicide, said method comprises
- obtaining at least one living organism,
- providing an assayable form of said part of living organism, said assayable form of living organism material comprising at least one group of chemical compounds,
- visualising said at least one group of chemical compounds optionally by a visual and/or UV-light detection,
- correlating said visualising of at least one group of chemical compounds to a standard visualising scale of said at least one group of chemical compounds,
assessing whether said living organism has developed resistance towards said pesticide.

By the term "living organism" is to be understood that the organism was living at least until the time where the living organism or a part of a living organism was collected to be utilised for a test according to the method described herein. A living organism may have visual sign of reduced growth e.g. some of the signs described elsewhere herein, although also other sign of not being a healthy organism may also be present on a living organism. In respect of plants a living organism is to be understood as all plant parts not being recognised as dead.

By "obtaining, providing or selecting at least one living organism" it is to be understood that also parts of this living organism can be used in respect of organisms where this is possible. In respect of plants, the test may be developed with plant parts and the testing can be performed with only a part of a living organism.

In an embodiment the method prior to visualising the at least one group of biochemical compounds further comprises,
- providing at least one chemical reagent applicable for a chemical reaction with said at least one group of chemical compounds, and
- provoking a chemical reaction between said chemical reagents and chemical compounds of said living organism material, and
where said visualising of at least one group of chemical compounds is a detection of a result of said chemical reaction.

If it is not known whether the plants to be tested for the presence of resistance are treated with herbicides, it is also possible to perform the test in two steps:
1. Performing an analysis which results in a colour of the extract e.g. reacting plant extract with a chemical compound as described elsewhere herein and obtaining a colour of the reacted extract. Based on the colour it is possible to determine whether the plant is resistant or it is a sensitive plant which has been exposed to the herbicide. If the result indicates that the plant is resistant it is possible to check whether this is true by performing a second step:
2. An analysis based on reacting plant extract with another chemical compound than the one used in step 1. Hereby it is possible to determine whether the plant is a resistant plant and not a plant which has not been exposed to the herbicide.

By "chemical reagent" is to be understood one or more chemical substance which can react with one or more chemical compounds of the living organism. The chemical reagent may perform a single chemical reaction with one or more chemical compounds of the living organism, or multiple chemical reactions may be performed, either simultaneously or following each other. The chemical reagent(s) may be provided as a single solution or a single dry matter e.g. as a salt or powder and/or as more than one solution and/or more than one dry matter. Preferred is chemical reagent(s) provided as a single solution or a single dry matter.

The chemical substances may each or all be dissolved in at least one solvent e.g. an organic solvent. Different chemical substances may be obtained in different solvents.

The at least one living organism may be a population of a living organism. The at least one living organism may also be one or more living organisms from a small group of individuals located in an area. The at least one living organism may also be one or single organisms living in an area, and where the number of said living organism in said area need not be considered to consist a population.

The living organism can be selected from but is not limited to the group of plants, fungi and animals. Also living organism such as insects, bacteria, mites, nematodes, rodents and vira can be tested according to the present invention.

Living organisms especially develop resistance against chemical compounds such as pesticides. Pesticides can be selected from the group of herbicides, insecticides, fungicides, bactericides, miticides, nematicides, rodenticides and virucides. Living organisms may, however, also develop resistance to other stress factors than pesticides such as drug resistance and antibiotic resistance, e.g. the ability of a microorganism to withstand the effects of antibiotics; Resistance to anitiviral drugs e.g. the ability of a population of viruses to withstand the effects of an antiviral drug.; Immune system, the system in organisms that provides disease resistance; Disease resistance in fruit and vegetables; Systemic acquired resistance, a response in plants that occurs following exposure to a pathogen.

Throughout this document the invention is illustrated by plants as living organisms and herbicides as the stress inducing factor. It is to be understood that plant and herbicide can be substituted with other living organisms and other stress inducing factors, respectively.

In an embodiment the invention further relates to a method for testing pesticide effects in plants, comprising the step of:
- providing at least one living plant, optionally treated with a pesticide,
- obtaining material from said at least one living plant,
- providing an assayable form of at least a part of said living plant,
- providing chemical reagents for a chemical reaction with a group of phytochemical compounds of said plant material,
- detecting a chemical reaction obtained by contacting said plant material and said chemical reagents,
- correlating said chemical reaction (colour and intensity) obtained on a solid support e.g. on a stick or disk to a standard result scale,
- assessing whether said living plant organism has developed resistance towards said pesticide.

The terms "standard result scale", "standard biomarker scale/pattern", "standard colour scale/pattern" and "standard visualising scale" can all be used to describe a standard scale or pattern obtained from a number of similar or nearly similar living organisms (e.g. biotypes of plants) which are subjected to the same level of stress, to different levels of stress e.g. to the same or different levels of a pesticide. The scale indicate the responses obtained within the living organisms in respect of amendments in production of different biochemical compounds including production of new biochemical compounds when compared to non-stressed living organisms or when compared to organism sensitive to the stress. By similar or nearly similar living organisms is meant that these living organisms need not be a variety or a clone, but genetic variety is accepted e.g. the living organisms may be biotypes of plants. Examples of similar or nearly similar living organisms may be a population of a plant species e.g. of a weed species; a population of a fungus e.g. species of a pathogenic fungus. Further examples of living organism to be analysed for the development of a standard scale is a number of biotypes of the specific organism e.g. at least two biotypes of a plant species. To obtain a standard result for the sensitive biotype, at least one sensitive biotype should be included when developing the standard scale.

A standard scale to be used to determine whether a living organism has developed resistance, can be developed by testing or analysing chemical compounds in different biotypes of the living organism. The different chemical responses obtained from the biotypes may correlate to differences in the response to pesticides.

The term "biotypes" is to be understood as subspecies or subgroups of organism which are morphologically similar to but physiologically different from other members of the species i.e. physiologically different from other biotypes of the species. A biotype is thus a strain of a species or variety that has certain biological characters separating it from other individuals of that species.

The number of biotypes used to develop a standard scale for a specific plant species in respect to a specific herbicide may be at least two biotypes, such as at least three biotypes, e.g. at least four biotypes, such as at least five biotypes, e.g. at least six biotypes, such as at least seven biotypes, e.g. at least eight biotypes, such as at least nine biotypes, e.g. at least ten biotypes, such as at least 12 biotypes, e.g. at least 14 biotypes, such as at least 16 biotypes, e.g. at least 18 biotypes, such as at least 20 biotypes, e.g. at least 22 biotypes, such as at least 24 biotypes, e.g. at least 26 biotypes, such as at least 28 biotypes, e.g. at least 30 biotypes.

It is preferred that at least one of the biotypes used when developing the standard scale is a sensitive biotype. The sensitivity to a specific herbicide of each biotype can be tested by determining the amount of herbicide needed to reduce the biomass to 50% of the non-treated plants of the same biotype. When comparing sensitivity among biotypes the "resistance factor" can be used to characterise the sensitivity.

The "resistance factor" is used to characterize the extent of susceptibility and resistance of a species to a herbicide. Resistance factors are typically represented as a ration of ED₅₀ values for different biotypes. ED₅₀ values are used to describe the amount of herbicide required to reduce a parameter of growth by 50%. The resistance factor therefore expresses the increase in herbicide dose required to reduce a growth parameter by half in a resistant compared to a susceptible biotype, and hence is a quantitative measure of the degree of resistance

The method of the present invention can be based on visualising, identifying or measuring a pooled or non-separated group of chemical or phytochemical compounds obtained e.g. in extracts from a living organism or plant where this non-separated group of chemical compounds is identified by a single signal e.g. by a single spot and/or a single colour. The colour may be determined visually on a solid support e.g. a stick or disk. Different colours reflect different biomarker pattern of the organism and thus indicate the difference in the organism's sensitivity to the stress such as the sensitivity of plants to a herbicide.

The group of biochemical compounds in the living organism can also be separated into sub-groups and/or individual compounds of the group and these sub-groups and/or individual compounds become part of the visualised, identified or measured result, hereby e.g. a number of coloured spots may visualise the chemical compounds of the living organism. Hereby the biomarker pattern of the plants are visualised by the results obtained e.g. as a colour pattern on a solid support, or as amount of different groups and/or sub-groups of different chemical compounds of the organism e.g. phytochemicals of plants. By comparing, e.g. a colour pattern or another pattern obtained in respect of a single plant or a group of plants with a standard colour pattern or another pattern, it is possible to determine whether the plant or group of plants have developed resistance to a specific herbicide.

In a preferred embodiment a chemical reaction is performed with the group of chemical compounds obtained from the living organism. Preferred is also that this chemical reaction is performed before visualisation, identification or measurement of the chemical compounds obtained from the living organism either as a non-separated group or as a separated group. The chemical reaction may be performed directly on crushed material from the living organism or may be performed with extract obtained when filtering crushed material. The chemical reaction may be performed before the extract or the crushed material is contacted with a solid support or after the extract or the crushed material is contacted with a solid support.

When the assayable form of a living organism is crushed material or an extract this may include chemical or phytochemical compounds which are assayed together in one step without separating the group of compounds into individual compounds. One or more groups of chemical or phytochemical compounds obtained from a living organism such as in a crushed material or extract and which are not separated into subgroups of compounds or into individual compounds may be denoted "pooled chemical compounds", "non-separated chemical compounds" or "gathered chemical compounds". "Chemical" may be replaced by "phytochemical" when the chemical compounds are obtained from or present in plants.

The non-separated biochemical compounds obtained from a living organism may depending on the actual amount and types of chemical compounds have some unique characteristics, which may be used to determine the level of stress impose upon as well as the resistance of the living organism. Any usable detection method may be used to distinguish between different samples of non-separated chemical compounds or of separated chemical compounds.

To simplify the description of the present invention the following text will concerns material for which the living organism is exemplified by plants and the method of detection the non-separated or separated phytochemical compounds is exemplified by colour reaction which can be detected in visual light or in ultra-violet light (UV-light). The term "chemical compounds" or "biochemical compounds" is exemplified by phytochemical compounds. Further, the invention particularly discloses the use of phytochemical compounds in plants to detect resistance in these plants towards herbicide, although resistance of other living organism and to other chemicals can also be tested by the present invention.

When the non-separated or separated phytochemical compounds are obtained from a plant, these may be reacted with a chemical reagent and a colour reaction may be detected either in visual light or in UV-light and semi-quantified using the intensity of the colour. In a preferred embodiment the colour is visualised on a solid support. In a more preferred embodiment the colour is visualised on a stick or a disk made of a material which may retain the phytochemical compounds optionally together with liquid comprising plant extract, solvents and/or a chemical reagent.

The colour and intensity of the reacted phytochemical compounds when applied on the solid support is different depending of the stress imposed upon the plant e.g. dependent on the dose of a pesticide or herbicide and/or the colour is dependent on the sensitivity of the plant to a pesticide. The colour and colour intensity can be correlated with a standard colour or visualising scale to determine presence of resistance. Extract obtained from a plant not exposed to a stress such as pesticide/herbicide has a different colour and/or colour intensity when compared to extracts from pesticide/herbicide exposed plants.

A scale of colours and colour intensity obtained e.g. on a solid support may be obtained when testing for and/or analysing groups of phytochemicals obtained from different plants of similar type e.g. of different biotypes which respond differently to a pesticide. The colour scale obtained based on phytochemicals extracted from plants from different biotypes indicate the presence of resistance. The extract from different biotypes can be distinguished on a solid support due to different colours and/or different colour intensity. The tests may be developed to the use of non-separated or separated phytochemicals from the plant.

By non-separated phytochemicals it is to be understood that substantially all groups of phytochemicals present in a plant part to be tested are also present in the plant material tested e.g. in crushed plant material or in extract obtained from crushed plant material. When performing a simple filtration of crushed plant material an extract is obtained. This extract is considered to comprise non-separated phytochemicals, as it is expected that no groups of phytochemicals are lost or disappear from the material during the crushing and filtration processes. A simple filtration may be a filtration through filter paper, such as e.g. a nitrocellulose filter or Whatman paper.

In an embodiment the invention relates to a method for testing pesticide effects in plants, comprising the step of:
- providing at least one living plant, optionally treated with a pesticide,
- obtaining material from said at least one living plant,
- providing an assayable form of at least a part of said living plant, said assayable form comprising non-separated or separated phytochemicals;
- providing at least one chemical reagent for a chemical reaction with one or more groups of phytochemical compounds known to be present or expected to be present in said non-separated or separated phytochemicals from the plant material,
- detecting at least one chemical reaction obtained by contacting a volume of said plant material and said at least one chemical reagent,
- correlating said chemical reaction (colour and intensity) obtained on a solid support e.g. on at least one stick or disk to a standard result scale provided in respect of said non-separated or separated phytochemicals,
- assessing whether said living plant organism has developed resistance towards said pesticide.

The present invention is based on the recognition that the phytochemical compounds in plants exposed to stress, such as pesticides, are related to and depending on the pesticides used and their modes of action in the plant. The inventor has found reproducible and unique colour reactions of separated and non-separated composition of groups of phytochemical compounds in plants after exposure to a stress factor, such as a pesticide, said colour reactions being unique to the specific stress factor and level of applied stress, and unique to the individual plant family, more preferred the individual plant species, or biotypes of plants. The unique colour reaction may be regarded as a fingerprint of the effect of a specific pesticide in the plant in question, i.e. the specific plant to be tested. Thus, the present invention offers an opportunity to assess/determine whether a plant has developed resistance to a pesticide.

Certain new chemical compounds may be produced in the plant after exposure to stress, or the concentration of already existing compounds may change, for example by an accumulation of certain chemical compounds in the plants. Furthermore, the colour obtained based on non-separated or separated chemical compounds may also be related to a decrease or even an elimination of chemical compounds in the plants after exposure to stress. These changes of concentration of compounds, elimination of compounds and/or production of new compounds after stress exposure may be due to changes in the biochemical pathways of plants.

Accordingly, a colour obtained based on a single group of non-separated chemical compounds is a unique fingerprint of the composition of phytochemical compounds, i.e. endogenously produced compounds, in the plant after exposure to a stress factor, i.e. an external exposure, and said fingerprint is unique for each type of stress factors, such as pesticides, or for a group of stress factors.

Also colours obtained based on at least one group of phytochemicals of separated chemical compounds is a unique fingerprint of the composition of phytochemical compounds in the plant after exposure to a stress factor, and said fingerprint is unique for each type of stress factors. Separation of phytochemicals may be into some or all of the groups of phytochemicals present in the plant material. The groups of phytochemicals may be selected from the groups of organic acid, lipids , reducing compounds, phenolic compounds, general compounds, amino acids, aromatic amino acids, N-compounds, alkaloids, amines, flavonoids, fytosterols, ketoses, glucolipids, cationes, diazepines, aldehydes, carbohydrates, glycosides, lipids, phospholipids, steroids and/or S-compounds.

The phytochemical compounds can also be associated to the chemical compound or parts of the chemical compounds used as chemical stress to the plants e.g. decomposition of the chemical compound to be detoxificated of the plants by a reaction with the reactive groups of the phytochemical compounds.

In one aspect of the invention, the compounds present in the plants after exposure are the same as before exposure, but the concentration of the individual compounds is different, whereby a new fingerprint of the phytochemical compounds has arised after exposure.

In an aspect of the invention the presence of phytochemical changes and the extend of sensitivity of the plant to stress exposure may be dependent on the age of the plant. Young plants tend to be more sensitive to exposure of stress, such as herbicides, than older plants. This means that a fingerprint of the phytochemical compounds can be detected at an earlier stage after the time of exposure in a young plant as opposed to the later stage of detection of a fingerprint of the phytochemical compounds in an older plant. Due to their high sensitivity young plants show lower stability of the biochemical changes, i.e. the fingerprint of the phytochemical compounds is more stable in older plants and may be observed throughout the remains of the life of the older plant. However, younger plants have a higher sensitivity to stress and also a higher mortality rate. Fewer species of young plants will survive stress exposure the first weeks after emergence, while older plants are less affected.

Accordingly, the present invention takes advantage of a number of parameters, such as the biochemical or phytochemical responses and the time after stress exposure with which they occur, the physiological effects, the types, numbers and concentrations of compounds biosynthesised in plants after exposure to pesticides.

In one embodiment of the invention the fingerprint of the phytochemical compounds of the plant composition may relate to one group of phytochemicals, such as to at least 2 groups of phytochemicals. In another embodiment of the invention the fingerprint of the phytochemical compounds of the composition relates to at least 3 groups of phytochemicals, such as at least 4 groups of phytochemicals, for example at least 5 groups of phytochemicals, such as at least 6 groups of phytochemicals, for example at least 7 groups of phytochemicals, such as at least 8 groups of phytochemicals, for example at least 9 groups of phytochemicals, such as at least 10 groups of phytochemicals. The groups of phytochemicals may be determined with or without a chemical reaction as described elsewhere.

By the term "standard colour scale" is meant a colour scale of the composition of compounds present in different plants e.g. different biotypes representing different genetic constitution of the plants and hereby possible different sensitivity to pesticides. According to the invention the fingerprint relating to one or more groups of phytochemical compounds of a plant or biotype to be tested for presence of resistance, i.e. the above described colour response of this plant or biotype is correlated to a standard colour scale. In order to interpret the fingerprint relating to phyto-chemical compounds of test material that has been exposed to pesticide, it is a prerequisite to provide standard colour scales. The colour reaction or fingerprint relating to phytochemical compounds of test material may then be correlated to standard colour scales. The standard colour scales may be obtained for one particular stress factor or for a combination of at least two different stress factors.

It is possible to prepare a standard visualising/colour/biomarker scale for material from a living organism comprising the steps of:
- subjecting at least one living organism to known types and known amounts of a pesticide or to no pesticide,
- obtaining material from said living organism,
- determining the chemical responses of said material from said living organism for each pesticide type and/or for each amount of pesticide, and
- obtaining at least one standard result relating to said pesticide type and/or to said amount of pesticide.

It is also possible to prepare a standard visualising/colour/biomarker scale for material from a living organism by using different biotypes of the living organism comprising the steps of:
- obtaining biotypes of at least one living organism e.g. from different living areas, said living areas optionally subjected to treatment with pesticide,
- obtaining material from said living organism,
- determining the chemical responses of said material from said living organism for each biotype, and
- obtaining at least one standard result relating to said pesticide and to the resistance of said biotypes of living organism.

In a preferred embodiment the chemical response of a living organism is based on compounds in the living organism which are correlated with occurrence of resistance in said living organism.

The description herein applies to both a method of providing a standard colour scale relating to phytochemical compounds of plants exposed to pesticide treatment which has different effect on the plants as well as to a method of testing whether material from a living organism has developed resistance to the pesticide.

The material on which the testing is performed may be from any living material, such as from animals, for example mammals, soil invertebrates and insects, or from thallophytes, such as fungi or algae. However, in a preferred embodiment of the invention the material from a living organism is plant material.

In another preferred embodiment the material is selected from plants, fungi or algae.

The following is a description of one embodiment of the invention, wherein the material from a living organism originates from plants. The description of this embodiment of the invention using plants, also relates to other embodiments of the invention, wherein the material from a living organism is not plant material.

Thus in one embodiment of the invention the method of testing is to determine the chemical fingerprint relating to phytochemical compounds after exposure to pesticide to test for presence of resistance in the plant. Based on the testing of different biotypes and a selection of which group or groups of phytochemical compounds the test has to be developed in respect of, the standard scale is developed giving a scale of different fingerprints or different colour pattern or different colours or different colour intensity, each indicating the sensitivity of the plant material.

The method and test kit according to the present invention can be applied to detect resistance to pesticides in e.g. weeds, pathogenic fungi and pests in agricultural and horticultural crops.

Plants react to stress exposure from herbicides. A change in the phytochemical composition and the concentration of the compounds in plant (a biomarker pattern) occur when plants are exposed to herbicides

Resistant individuals are typically from 3-4 times to 1000 times less sensitive in respect of a pesticide when compared to sensitive individuals. Hereby an insufficient effect is obtained of a pesticide treatment when treating with a recommended dose and the resistant organisms will survive the treatment. It has turned out that resistant biotypes are not affected by herbicides at least not when it comes to the reduction in biomass, and significant differences in the biomarker pattern between susceptible and resistant biotypes can be obtained.

When using the method and the test kit a measurement of the phytochemical (biochemical) response of organisms surviving a pesticide treatment is performed. By comparing this response to the standard response of susceptible and resistant biotypes of that specific weed, pathogenic fungus or pest it is possible to decide whether an unsatisfactory response can be related to pesticide resistance or is caused by other factors. The standard biomarker pattern of the test kit can be developed using a collection of susceptible and resistant biotypes.

The composition and/or the content of natural compounds in susceptible or resistance biotypes of the organisms are different. This is also the instance with organisms exposed to chemicals. By comparing the content and composition of the natural chemical compounds in the organisms, it is possible to detect differences which are used to identify the biotypes. The differences in the composition and the content of natural compounds within the different biotypes which are exposed and unexposed with chemicals are used to define the overall natural chemical compounds to be used to prepare a standard biomarker/visualising scale and to develop the test-kit as described herein.

Histochemical methods can also be used to detect the differences in natural chemical compound composition and/or content in the different biotypes. Different parts of the organisms e.g. all different parts of the plants: stem, leaf, petals etc. can be used to detect the natural- or phytochemical compounds directly by microscope before i.e. without or after a chemical treatment to perform a chemical reaction. The parts of the organism can be used directly or cut into small pieces e.g. slides. A chemical reaction between chemical compounds of the organism and a chemical reagent can be detected as a colour reaction in white light or by UV-light with or the chemical compounds of the organism i.e. the biomarkers of the organism can be detected without using chemicals.

In a preferred embodiment the test is a qualitative assay providing the user with a yes or no answer to the question whether an unsatisfactory effect is obtained following the use of a pesticide dose generally expected to kill the specific weed, pathogenic fungus or pest. Hereby the test is developed to distinguish between chemical compounds i.e. biomarker pattern from organism which will die due to the chemical treatment and organism which will survive the chemical treatment.

The test can detect both target-site resistance, i.e. resistance caused by a mutation resulting in an insensible target site, and metabolic resistance, i.e. a mutation enabling the weed, pathogenic fungi or pest to metabolise the pesticide faster than possible for susceptible biotypes.

Specific test kit can be developed for each case of pesticide resistance, i.e. for example for each combination of herbicide and weeds, fungicides and pathogenic fungi and insecticides and pests. If different resistance mechanisms are found in the same weed, pathogenic fungus or pest different test kits may be developed. Surviving weeds, pathogenic fungi or pests can be collected when it is obvious that these will survive a pesticide treatment and can be tested in accordance with the invention described herein. Certain pesticides, particularly insecticides but also some herbicides, have an immediate knock-down effect, hence surviving individuals can be collected already 1-3 days after treatment. For other pesticides, particularly herbicides and fungicides, symptoms develop more slowly and whether an individual will survive cannot be determined until 10-14 days after pesticide treatment. Time of collection may be specific for each combination of pesticide and weed/ pathogenic fungi/pest.

The plant material of the invention may be selected among any plant or plant cells. The plant material may be chosen from vascular plant, pteridophytes, seed plants, the gymnosperms, the angiosperms, mono- and dicotyledons. In one preferred embodiment of the invention the plant material is chosen from, but not limited to dicotyledons or monocotyledons. Also preferred is plant material chosen from plants considered to be a weed, especially weed in crop plants is of interest. Weed is considered as a plant competing with the crop plant such that the crop plant is negative influenced either in growth and/or composition.

Two types of herbicide resistance in plants have been documented. These are target-site resistance and enhanced metabolism.

Target-site resistance:
- The herbicide will not bind to the target site
- Frequently monogenic inherited
- High selection pressure will increase the proportion of resistant biotypes in the population
- Often cross-resistance to herbicides with the same mode of action.

Enhanced metabolism:
- Increased metabolism of the herbicide
- Assumed to be polygenic inherited
- Relationship between selection pressure and build-up of resistant biotypes in the population not well-established
- Often cross-resistance to herbicides with various modes of action.

Both of these resistance types can be detected with the method and kit of the present invention.

According to the invention the dicotyledonous plants may be selected from the families of Asteráceae, Brassicaceae, Lamiaceae, Polygonaceae, Papaveraceae, Primuláceae, Plantagináceae, Convolvolaceae, Umbelliferae, Oenotheraceae, Papilivanaceae, Violaceae, Malvaceae, Euphorbiaceae, Geraniaceae, Cruciferae, Fumariaceae, Urticaceae, Caryophyllaceae, Portulacaceae, Amarnthaceae, Cnenopodiaceae, Ranunculaceae, Boraginaceae, Labiatae, Solanaceae, Rubiaceae, Compositae and Scrophulariaceae and the monocotyledonous plants may be selected from the families of Poáceae/Graminea, Cyperaceae, Alismataceae, Lemnaceae, Potamogetonnaceae, Hydrocharitaceae, Juncaceae, Liliaceae, Convallariaceae, Iridacaea.

In a preferred embodiment the plant is selected from a plant of the genus's *Apera, Alopecurus, Lolium, Bromus, Setaria, Echinochloa, Stellaria, Papaver, Polygonum, Galeopsis, Sinapis, Amaranthus, Brassica, Tripleurospermum, Matricaria* and Poa.

In a further preferred embodiment the plant is selected from the group of plant species *Apera spica-venti, Alopecurus myosuroides, Avena fatua, Lolium perenne, Bromus hordaceus, Poa annua, Stellaria media, Tripleurospermum inodorum, Chenopodium album, Amaranthus retroflexus, Galeopsis sp., Papaver rhoeas, Lolium sp., Setaria sp., Echinocloa crus-galli* and *Conyza canadensis.*

Non-limiting examples of test kits developed for detection of resistance are:
- In weeds:
   ∘ Sulfonylurea herbicides and *Stellaria media, Galeopsis sp., Papaver rhoeas, Tripleurospermum inodorum, Avena fatua, Apera spica-venti, Alopecurus myosuroides, Lolium sp.*
   ∘ ACC'ase inhibitors and *Alopecurus myosuroides, Lolium sp., Setaria sp., Echinocloa crus-galli*
   ∘ Glycines and *Conyza canadensis, Lolium sp.*
- In pathogenic fungi:
   ∘ Strobilurines and *Mycosphaerella gramincolai, Blumeria graminis f. sp tritici* and *hordei* and *Dreshsiera tritica repentis (DTR)*
   ∘ Sterol biosynthesis inhibitors and *Mycosphaerella gramincolai, Blumeria graminis f. sp tritici* and *hordei*
- In pests:
   ∘ Organophosphates/carbamates and *Myzus persicae*
   ∘ Synthetic pyrethoids and *Meligetes aeneus*

According to the invention the plant material used to perform the method of testing may be the entire plant or it may be at least a selected area of any part of the plant. The selected area of the plant may be an area such as from at least flowers, shoots, leaves, stems, roots, seeds, pollen, rhizomes, stamens, sepals, petals, carpels, styles, stigmas, microsporangia, anther, fruits, cotyledons, hypocotyle, epicotyle, xylem and/or phloem (wood), periderm (bark), buds, flower buds, cones, cone scales, tubers, bulbs, root nodules, resin or sap, or a combination thereof.

Preferred is plant material obtained from a flower. Preferred is also plant material obtained from a shoot. Also preferred is plant material obtained from a leaf. Further preferred is plant material obtained from a stem. Yet further preferred is plant material obtained from a root. Also preferred is plant material obtained from a seed.

The test kit may be developed to test plants in stage or phase 12 (2 leaves) to stage or phase 23 (bushy). The stage/phase may also be 10, 13, 14, 15, 16, 17, 18, 19, 20,21,22,24,25,26.

Once a sample of the plant material is obtained, a second step in the method according to the invention begins. It is an object of the present invention to provide a method of testing, wherein the plant material used is in a form suitable for assaying. One such form may be a liquid form, for example a liquid suspension. A liquid suspension of the plant material may be obtained by applying extraction solvents, such as water or ethanol to the plant material. The solvent may ensure that all compounds from one or more chemical groups present in the plant material are extracted. The assayable plant material may be fresh or non-fresh.

In a preferred embodiment of the invention the plant material is fresh. The fresh material may be used for analysis immediately after harvest said material or it may be used for analysis up to a few minutes after harvesting. The fresh material can be analysed within at least 15 min, such as 30 min, e.g. 45 min, such as 1 hour, e.g. 2 hours, such as 3 hours. It is preferred that the fresh material is used as soon as possible after harvesting to avoid decomposition processes, such as enzymatic activity.

In one embodiment the plant material is frozen. The frozen plant material may be frozen up to the point of analysing, such as frozen for a period of at least 5 years and it may be defrosted prior to performing the test. However, it is preferred that the frozen plant material is used for analysis immediately after being removed from the cold storage. Any freezing process can be used to freeze the plant material. Preferred is when the plant material is subjected to the freezing process immediately after harvest, e.g. within 5 min, such as within 15 min, e.g. within 30 min, such as within 45 min, e.g. within 60 min, such as within 75 min, e.g. within 90 min, such as within 105 min, e.g. within 120 min.

In another embodiment of the invention the plant material is dry. The drying process may be accounted for by air, or nitrogen, or it may be a freeze drying process, such as nitrogen dried. Additionally the plant material may be heat dried, such as sun dried. The plant material may be substantially dry, and the length of the drying process is dependent on the type of plant material. Air drying may be at about 20°C and e.g. without heat and light. Heat and light might destroy the compounds of the material.

The length of the time period before the plants react to the pesticide exposure and sensitivity of the plant species to the pesticide may be dependent on different factors, such as the species and age of the plant. The various plant species have different sensitivity to pesticide types. For example the plant species *Lolium perenne* is more sentitive to the sulfonylurea herbicide, iodosulfuron than *Apera spica-venti* or *Poa annua.* Therefore a lower dose of the herbicide exposed to *Lolium perenne* than to *Apera spica-venti* or *Poa annua,* may be detected as a phytochemical response corresponding to a higher reduced biomass to *Lolium perenne* than to *Apera spica-venti* or *Poa annua.* With respect to age, the seedlings may be more sensitive than older plants to the herbicides and therefore seedlings are more sensitive to the herbicide.

In an embodiment the method and kit is developed in respect of a living organism mentioned elsewhere herein at at least one development stage selected from the principal growth scales 0, 1, 2, 3, 4, 5, 6, 7, 8 or 9 representing stages of germination, sprouting, bud development, leaf development, formation of side shoots/tillering, stem elongation or rosette growth, shoot development (main shoot), development of harvestable vegetative plant parts or vegetatively propagated organs/booting (main shoot), inflorescence emergence (main shoot)/heading, flowering (main shoot), development of fruit, ripening or maturity of fruit and seed, senescence, beginning of dormancy. Growth scales are further described in "Growth stages of mono- and dictolydonous plants", BBCH Monograph, 2. edition 2001. Edited by Uwe Meier, Federal Biological Research Centre for Agriculture and Forestry.

In an embodiment the method and kit is developed to be useable on a plant species or variety within a time period lasting for at least more than the time the organism has to growth within one growth scale. Hereby the method and kit can be developed to be used in e.g. growth scale 0 and 1 (partly or fully), or growth scale 1 and 2 (partly or fully); growth scale 2 and 3 (partly or fully); growth scale 3 and 4 (partly or fully). Also more the two growth scales may be covered by the method and kit, e.g. growth scale 0 to 2 (partly or fully), growth scale 1 to 3 (partly or fully), growth scale 2 to 4 (partly or fully), growth scale 0 to 3 (partly or fully), growth scale 1 to 4 (partly or fully), growth scale 0 to 4 (partly or fully).

The detection of phytochemical effect may be possible as long as the plant is living. This may be between less than one day and up to 21 days after exposure, such as between 1-20 days after exposure, for example between 4-7 days after exposure.

In an embodiment the method is developed to be used to test plant material before or without exposure to a herbicide or at least 1 day after exposure, e.g. at least 2 days, such as at least 3 days, e.g. at least 4 days, such as at least 5 days, e.g. at least 6 days, such as at least 7 days, e.g. at least 8 days, such as at least 9 days, e.g. at least 10 days, such as at least 11 days, e.g. at least 12 days, such as at least 13 days, e.g. at least 14 days, such as at least 15 days, e.g. at least 16 days, such as at least 17 days, e.g. at least 18 days, such as at least 19 days, e.g. at least 20 days, such as at least 21 days, e.g. at least 22 days, such as at least 23 days, e.g. at least 24 days, such as at least 25 days, e.g. at least 26 days, such as at least 27 days, e.g. at least 28 days, such as at least 29 days, e.g. at least 30 days, such as at least 35 days after exposure.

In an embodiment the method of testing for resistance relates to all pesticides representing groups with different mode of action. For example glyphosate and glyphosate like herbicides or sulfonylurea herbicides.

The detection of a fingerprint in a plant may in one embodiment of the invention serve the purpose of an "early warning" signal of initiation of resistance development in the plant before it is reported that a certain pesticide has lost its effect.

It has been reported that when plants are exposed to stress they may react by changing their phytochemical (biochemical) composition. The present invention presents a method by which reproducible fingerprint relating to phytochemical compounds are obtained, thus providing analytical tools for the establishment of exposure to and identification of known as well as unknown compounds. There is a variety of stress factors that may all have an impact on the chemical composition of plants. The plant may be exposed to more than one stress factor, wherein in one embodiment the effect of the exposure is synergistic and thus results in a fingerprint relating to phytochemical compounds reflecting the synergistic effect of the individual stress factors. In another embodiment, wherein the plant may be exposed to more than one stress factor, the resulting fingerprint relating to phytochemical compounds reflects the antagonistic effect of the individual stress factors. It is within the scope of the invention to develop a standard fingerprint relating to phytochemical compounds for any combination of stress factors such as for combinations of pesticides e.g. combinations of herbicides.

The plant species used in the invention may be treated with at least one herbicide, such as at least two herbicides, e.g. at least three herbicides, such as at least four herbicides, e.g. at least five herbicides, such as at least six herbicides, e.g. at least seven herbicides, such as at least eight herbicides, e.g. at least nine herbicides, such as at least ten herbicides. The herbicides used may be any suitable for treating the plant species, or may be selected among the herbicides mentioned herein, such that any combination of the mentioned herbicides in the numbers given above is disclosed.

According to the invention one of the stress factors is abiotic, such as chemical stress and/or physical stress.

In the present context chemical stress may be caused by pesticides, such as herbicides. Herbicides are all designed to kill plants by altering and affecting the bio-chemical homeostasis of the plant cells. Plants react to the exposure of herbicides by producing or decomposing phytochemical compounds. They may also react by changing the concentration of already existing compound(s). The resulting effect on the plants is dependent on the individual mode of action of the herbicide.

In an embodiment of the invention the method of testing for the exposure of pesticides relates to herbicides and/or pesticides comprising active ingredients selected from the group consisting of sulfonylurea herbicides, ACC'ase inhibitor herbicides, glycine herbicides, strobilurine fungicides, sterol biosynthesis inhibitor fungicides, organophosphate insecticides, carbamate insecticides and synthetic pyrethoid insecticides or a combination thereof.

In another embodiment the method is for testing for resistance to azoxystrobin (strobilurine fungicides) or pirimicarb (carbamate insecticide).

The pesticide/herbicide can also be selected from the group consisting of Glypho-sate, Bromoxynil, Pendimethalin, Metsulfuron methyl, Prosulfocarb, Clodinafoppropargyl, Fenoxaprop-p-ethyl, lodosulfuron, Mesosulfuron, Sulfosulfuron and Flupyrsulfuron or a combination thereof.

The active ingredients may all represent different modes of action on the target plants. Pesticides/herbicides with the active ingredients described above are all widely used in Northern America and Western Europe for the control of e.g. broad-leaved plants and grasses. Other pesticides than the ones mentioned above are also within the scope of the invention. They may be the ones described in The Pesticide Manual, British Crop Protection Council. For example insecticides, acaricides, nematicides/vermicides, rodenticides and fungicides may be the stress inducing factors.

Glyphosate (GLY) is a non-selective herbicide that controls emergent annual and perennial broad-leaved plants and grasses. Glyphosate inhibits the activity of the EPSP-enzyme (5-enolpyruvylshikimate-3-phosphate) of the aromatic acid biosynthetic pathway in plants. It is absorbed through the wax cuticle on the leaves and a rapid translocation occurs via phloem to roots, rhizomes and apical meristems. It is degraded by rapid microbial action, with a half-life of 3-5 weeks. It is non-volatile and does not degrade photochemically. The water solubility is 11.6 g/l at 25 °C. It binds strongly to soil particles and hereby it is immobile unless transported with the soil.

Bromoxynil (BRY) is a selective herbicide with some systematic activity. The herbicide is absorbed by the foliage through cuticular penetration. Bromoxynil kills by inhibition of photosynthesis and plant respiration in annual broad-leaved plants. It degrades rapidly in most soil types, with a half-life in the order of two weeks which can be considerable reduced at low temperatures. It is water-soluble (130 mg/l), potentially harmful to fish and aquatic invertebrates for which it is toxic if it reaches water bodies.

Pendimethalin (PEN) is a selective herbicide that inhibits cell growth by inhibiting cell division of any and all plant cells by acting as a mitotic toxin. It is absorbed by roots and leaves, but initially limits root growth, such as the development of lateral or secondary roots. Pendimethalin is moderately persistent in moist sandy loam (half-life 50 days) to highly persistent in moist silty soil (half-life 140 days) and in dry silty clay loam (250 days). It is a very stable herbicide except when it volatilises from moist soil surfaces. The water solubility is 0.3 mg/l at 20 °C. Thus, it is likely to be transferred to other environmental compartments although it may move with soil particles to water bodies where it is toxic to fish.

Metsulfuron methyl (METS) is a potent inhibitor of plant growth used on wheat and barley crops for the control of broad-leaf species and the suppression of few grasses. The herbicide is taken up by the foliage or the roots and translocated via xylem and phloem. Metsulfuron methyl is a selective herbicide that acts by inhibiting the enzyme acetolactate synthase (ALS) which catalyses the synthesis of the three branched-chain amino acids valine, leucine and isoleucine. The precise mechanism of action is unknown, but soon after herbicide application, plant cell division quickly stops, and death occurs within one to three weeks. The accumulation of ALS substrates (e.g. α-ketobutyrate) in leaves may be responsible for the cessation of the plant growth with decreased production of new leaves and reproductive organs. Metsulfuron methyl is mobile in most soil and the mobility is enhanced as pH increases.

All the above mentioned herbicides are currently applied to major crops, such as maize, wheat, barley, soybeans, oats, peas, potatoes and tomatoes. When applying herbicides to a cultivated field of crops adjacent non-target areas may be affected by herbicides as well. The present invention may be used to test whether weeds or pathogenic fungi or pest e.g. in insect pests in crops treated with pesticide have developed resistance. The invention may also be used to test whether plants in a non-target area are affected by a treatment in adjacent cultivated field and thus resistance is developed or to test if a conventional crop has been polluted with a genetically modified herbicide tolerant crop.

Further, in the present invention the method of testing can be applied to plants potentially being exposed to and developing defence mechanisms to physical stress, such as temperature, wind, UV light, physical damage, soil quality and soil moistness, salt etc.

In another embodiment the stress factors may be biotic, such as biological stress and/or allelopathy. The term "biological stress" is meant as stress and possibly visual damage caused by herbivores, plant pathogens and/or competition from other plants. The latter may also be referred to as allelopathy, such as competition from other plants and/or chemical compounds of other plants effecting/stressing the plant on which a test is performed to detect whether the plant has changed in sensitivity to this biological stress e.g. become less influenced.

The term "phytochemical" as used herein relates to any chemical or compound or nutrient or fundamental compound present in the plants. There are a vast number of compounds present in plants. Some of the compounds are readily detectable under circumstances where the plants are not exposed to pesticides. If, however, plants are exposed to pesticides the biochemical pathways within the plant cells may be affected. The influence of pesticides on biochemical pathway may lead to an increase or change, such as elimination in the concentration of already existing compounds, or it may lead to the production of compounds not normally present in plants not exposed to pesticides.

In an embodiment of the invention the composition of phytochemical compounds of at least one type and/or group is determined.

In one embodiment of the invention the phytochemical is a substance, or at least part of a substance, or a derivative of the groups amino acids, amines, sugars, flavonoids, phenolic compounds, sapogenins, saponins, iridoids, glycosides, alcaloids, alkaline alcaloids, C-containing compounds, N-containing compounds, S-contaning compounds, P-containing compounds, O-containing compounds, any fundamental elements, terpenoids, lipids, steroids, cartenoids, quinones, coumarines, and nutrients, such as any compound necessary for the plant to survive, for example salts.

In another embodiment at least two of the mentioned groups are reacted simultaneous in one sample of extract or with different samples of extract for preparing a standard colour scale and for performing the test. Non-limiting examples of two groups are: amino acids and amines, sugars and flavonoids, phenolic compounds and sapogenins, saponins and iridoids, glycosides and alcaloids, alkaline alkaloids and C-containing compounds, N-containing compounds and S-contaning compounds, P-containing compounds and O-containing compounds, any fundamental elements and terpenoids, lipids and steroids, cartenoids and quinones, coumarines and amino acids, although any other combination is also to be understood as disclosed.

By the term fundamental elements is meant any compound depicted in the periodical system.

The chemical analysis of pesticides is very difficult when the presence of the pesticide in the environment is low. Furthermore, it is very expensive to perform chemical screenings for chemical compounds, such as pesticides and/or their decomposition compounds and/or adjuvants present in pesticides. By the present invention it is now possible to determine resistance to chemical compounds, such as pesticides by a simple and affordable method of testing.

In one embodiment of the invention the method of testing comprises the following steps:
- contacting an assayable form of plant material with a support for receiving at least a part of said plant material,
- subjecting said support to a solvent,
- optionally drying said support,
- optionally contacting said support with a chemical reagent,
- obtaining a fingerprint relating to phytochemical compounds of said assayable form.

The obtained fingerprint may be based on one or more separated and/or non-separated groups of chemical or phytochemical compounds. In a preferred embodiment the test is performed with non-separated groups of chemical or phytochemical compounds.

In the present context an assayable form may be a liquid, or a liquid mixed with solids, such as liquids mixed with salts. An assayable form of an organism can also be squeezed material optionally filtered to remove components from the squeezed material i.e. a example of an assayable form of the organism is a filtrate.

In another embodiment, the testing comprising similar steps as described above but the chemical reaction is performed before the assayable form of the plant material is contacted with a support, hereby the support need not be subjected to a solvent.

When testing by using one or more non-separated groups of chemical or phytochemical compounds the solid support can be partly or entirely embedded in an extract of the organism. Also the colour determination can be performed on a solid support which has absorbed part of or the entire volume of the extract or another assayable form of the organism.

Obtaining a fingerprint relating to phytochemical compounds may be in the form of a colour reaction on the support, such as one or more colour spots e.g. with different colours and/or different colour intensity.

In an embodiment the chemical reagent and/or solvent is based on one or more of the compounds selected from the group of chlorofenolred , methylred, ethylred, bromothymol blue, 2.6-dichlorophenolindophenole sodium salt, bromocresolpurpur, ninhydrine, vanillin + potassium hydroxide, glucose, 4-chloro-7-nitrobenzofurazan, 2.4-dinitrophenylhydrazine, 9-fluorenylmethylchloroformate, tetrabutylammoniumhydroxide, iode + potassium iodine, bismuth (III) nitrate, Ammonium ferri(III)sulphate, 2-methoxy-2.4-diphenyl-3(2H)furanon (MDPF), 2-aminoethyl-diphenylborinate, fer-ri(III)chloride, aluminium chloride, berberine chloride dihydrate, 1.2-naphthochinon-4-sulfonsodium salt, anthrone, 8-hydroxychinolin, 2-aminodiphenyl(biphenyl-2-amine), orcinol, urea, 4-hydroxybenzoic acid, 4-aminobenzoic acid, molybdatophosphoric acid, 2'.7'-dichlorofluoresceine, 8-anilinonaphthaline-1-sulfonic acid-ammonium salt, rhodamine, iod, potassium iodide, ammoniummolybdattin(II) chloride, cobalt(II) chloride, palladium(II) chloride,) nitrate potassium iodide, vanillin, sulphuric acid, naphtoresorcinol, methylene blue, β-naphtol, thymol, fluorescein, ammonia, bromocresol green, bromophenol blue, potassium permanganate, 2,7-dichlorofluorescein, rodamin 6G, diphenyl boric acid 2-aminoethylester, phosphoric acid, iod, potassium iodide, ammoniummolybdattin(II) chloride, cobalt(II) chloride, palladium(II) chloride, 1-naphthol, ninhydrin, bismuth(III) nitrate potassium iodide, molybdat phosphor acid, rhodamin B, (p-and o) anise aldehyde, silver nitrate, ferro(III) chloride, and zinkchloride and chemicals or mixtures hereof. Chemical reagent needed for the reaction is 0.001 - 10 mg/ml extract depending of the reaction mechanism, conditions and supplementary chemicals.

The support for receiving the material may be a solid material, solid support or a less solid material, such as a soft material, for example a liquid material. The support may be pretreated with a substance capable of promoting reactions when contacted with the plant material. Said reactions may for example be detected by visual, radioactive, fluorescent, or immunological methods. Preferred is when the solid support is made of a material suitable of function as filter paper, such as e.g. nitrocellulose or Whatman paper or another material capable of absorbing liquid parts of the assayable form of the organism.

In a preferred embodiment the solid material or solid support is in the form of a stick or a disk. Preferred is also a stick or disk made of a fabric capable of absorbing at least a part of the solution with the phytochemicals optionally reacted with a chemical reagent.

The solid support is optionally in the kit described elsewhere herein. The colour of the plant extract or extract reacted with a chemical reagent may be determined by placing a container with the extract and with or without a solid support within the container close to the standard colour scale or a standard result and determining the colour of the plant extract or extract reacted with a chemical reagent. The solid support may also be removed from the container before comparing the colour of the solid support with the colours of the standard colour scale.

The solid support can be used to contact the reacted extract, but can also be used to obtain a background with a standard colour behind a container containing a coloured sample from a plant, such as a coloured extract. When the solid support is used as a background colour e.g. a white colour this minimises the risk of an erroneous determining of the colour of the extract within a container.

In an embodiment the standard colour scale may be an integral part of the solid support or holding means comprising the solid support. The solid support may comprise a section for applying the extract or reacted extract of the material to be tested and another section of the solid support may comprise a standard colour scale. Holding means may be a cassette e.g. enclosing a solid support and a standard colour scale may be attached to the cassette and/or to the enclosed solid support.

The solid support may also function as a stick or disk which is placed within the container with the extract. The solid support may absorb part of or all the volume of the extract or may be immersed within the extract. The colour of the solid support or of the extract may be determined when the solid support has absorbed part of or all the extract or when the solid support is immersed within the extract, also the colour can be detected when a volume of the extract is absorbed onto only a part of the solid support. In the latter case the extract can be sucked up by the solid support e.g. by dipping the solid support into the extract or by applying extract on the solid support. By extract is meant raw extract from the organism to be tested, extract in a solvent e.g. in water or extract which has been subjected to a reaction e.g. a chemical reaction and/or a colour reaction.

In an embodiment the use of chemical reagents and solid support e.g. stick/disks to determine the presence of resistance in a living organism comprises the following steps:
- contacting an assayable form of said living organism e.g. plant material with at least one chemical reagent,
- providing a chemical reaction between the assayable form of material from the living organism and the at least one chemical reagent,
- contacting a solid support with the material from the living organism chemically reacted with the at least one chemical reagent, hereby
- obtaining a solid support with a colour (visual or detection in UV-light)
- comparing the colour and colour intensity with a standard visualising scale,
- evaluating of existence of resistance in said living organism.

The standard visualising scale is obtained as described elsewhere herein.

In the present context the term "solvent" is meant to cover one substance or a combination of two or more substances, wherein the solvent may be a combination of liquid and/or gas substances. A solvent may be a reagent, an eluent or an extraction media. The latter three may be in a solid or liquid physical state, or they may be in the form of a gas.

In one embodiment of the invention an extract of material from a plant is provided. The extraction may be performed under cold or warm temperatures, such as by the means of ultrasound, or stirring.

The extraction solvent may be any useable solvents. Non-limiting examples are water with or without solid liquid or gas chemicals deluted, alcohol, acid, ether, petroleum or a combination thereof. The solvents mentioned may be in any concentration, such as e.g. 5, 10, 15, 20, 25, 30, 40, 50, 60, 70, 80, 90 or 100%.

Examples of solvents are petroleum-ether solvents, 10 % acidic acid in 96 % ethanol, 75 % ethanol. The extraction may be performed on fresh or non-fresh plant material, e.g. on frozen material with or without a defrosting process before initiating the extraction process.

According to the invention the solvents and the support may have different polarities, such as between -0.1-10, for example between 2-8, such as between 4-6 as defined by Snyder, (1974).

In one aspect of the invention the biomarker pattern is detected by the use of commercially available techniques known to the skilled artisan, such as High Performance Liquid Chromatography (HPLC) or gas chromatography or mass spectrometry (MS), or a combination of analytical methods. For example a densitometric evaluation of thin-layer chromatograms using a densitometric scanner/videoscan may be employed. It is important that the same method of analysing is applied when detecting both the standard biomarker pattern, and the biomarker pattern resulting from the plant to be tested for existence of resistance.

In a preferred embodiment the biomarker pattern is detected as a colour system e.g. as a single colour of the extract or of the extract reacted with at least one chemical. Different reactions to a pesticide may be visualised by different colours and/or different shades of colour(s) of the extract or of the reacted extract.

In one embodiment of the invention the biomarker pattern is detected by the use of Thin Layer Chromatography (TLC). TLC is a well-known simple chromatographical separation method. The technical advantage of using TLC techniques compared to e.g. HPLC, is the visual colour reaction of the plant biomarker. Additionally, TLC is a cheaper analysis compared with other analytical analysis. In one embodiment of the invention the TLC method is circular.

Other relevant biomarker detection methods within the scope of the invention are infrared spectrophometry, spectrophometry, refractrometri, nuclear magnetic resonance, and electrophoresis. Radioactive compounds may be used in said methods. For example a "film" of radioactivity may be placed over a TLC-plate, whereafter a pattern of biomarkers emerge where the radioactive compounds are placed.

When using the method of Thin Layer Chromatography testing according to the invention, it comprises the following steps:
- contacting an assayable form of said plant material with a TLC-plate,
- subjecting said TLC-plate to a solvent,
- optionally drying said TLC-plate,
- optionally contacting said TLC-plate with a chemical reagent,
- obtaining a biomarker pattern of said assayable form.

In a further aspect the biomarkers are separated by the means of antibodies possible attached to support or antibodies in the solvent.

In order to verify the shapes of the finger print of the composition i.e. the change of the composition of the biomarkers or phytochemical (biochemical) changes, different TLC-plates, solvents and chemical reagents may be used. This results in different colour reactions, which may be visually inspected. The TLC-plates used in the test of the invention may be commercially available TLC-plates, and may be made from cellulose or silica gel. The types of TLC-plates chosen for the test are selected according to the plant species tested, and to the specific compounds of which it is desired to determine their existence or absence in the plant material.

Once the extract of the plant material has been placed on a TLC-plate the TLC-plate can be placed in a TLC chamber containing a chemical eluent. The eluent is absorbed by the plate material and this initiates the development of the test. The biomarker compounds react with the plate material and the eluent. All biomarkers have different affinity to the plate material and to the eluent. Thus, the biomarkers will appear in different positions on the plate material. The higher affinity the biomarkers have to the eluent the further they will migrate on the TLC-plate. The first step of "developing" the plate, i.e. the reaction of the plant material with the plate material and element may be a period of 120 minutes, such as 90 minutes, for example 60 minutes.

In one embodiment of the invention the solvent comprises the upper-phase of n-butanol and formic acid in a ratio of 2:1. The solvent may only be stable for up to 1 day and therefore has to be renewed daily.

In another embodiment the solvent comprises n-butanol, acetic acid and water in a ratio of (4:1:5). The solvent is stable for several days and is preferably stored in a cool place.

The TLC-plate may then be air-dried and a chemical reagent may be brought in contact with the TLC plate, such as by spraying. The type of reagent of the invention may vary according to the type of TLC-plate and the type of biomarker. As the reagent of the invention is applied to the dried TLC-plate a unique and reproducible colour reaction develops. According to the invention the colour reaction may confirm or reject the presence of specific biomarkers after exposure to stress, such as herbicides.

Accordingly, a purpose of the invention is to provide a method of testing, wherein the biomarker pattern is obtained as a result of the specific combination of parameters, such as said support, for example TLC-plates, said solvent and said chemical reagent.

An object of the present invention is to provide an assay kit for testing for resistance in a living organism, the kit comprising
- at least one solvent and/or reagent,
- at least one standard colour scale,
- at least one container/glass
- optionally at least one solid support (e.g. sticks and/or disks).

The solvent and/or reagent may be solvent and/or reagent as described elsewhere herein. The amount of solvent/reagent may be between a few drops e.g. withheld on a solid support or in a flask to 100 mL. Preferred is solvent/reagent volume less than 75 mL, e.g. less than 50 mL, such as less than 25 mL, e.g. less than 15 mL. Preferred is also solvent and/or reagent volume between 0.5 to 5 mL, such as 5-10 mL, e.g. 10-15 mL, e.g. 15-20 mL. The volume of solvent and reagent may be different. Thus preferred volumes may be selected for each solvent/reagent among the ones mentioned above.

Preferred volume of raw extract may be between 0.1 mL and 5 mL, such as between 0.15 mL and 4 mL, e.g. between 0.2 mL and 3 mL, such as between 0.25 mL and 2 mL, e.g. between 0.3 mL and 1 mL, such as between 0.35 mL and 0.8 mL, e.g. between 0.4 mL and 0.5 mL.

An extract may be obtained by extracting an amount of plant material in a solvent, the ratio between the weight of plant material and the volume of solvent may be between 1:100 and 1:1, such as at least 1:80, e.g. at least 1:60, such as at least 1:40, e.g. at least 1:30, such as at least 1:25, e.g. at least 1:20; such as at least 1:15, e.g. at least 1:10, such as at least 1:5, e.g. at least 1:2. An example illustrating the described ratio is 0.2 mg plant material extracted with 3.5 mL of solvent.

The extract may be further diluted with the same solvent as used for extraction or with another solvent. The final ratio between the weight of plant material used initially for the extraction and the total volume of solvent optionally before performing any further reaction of the extract may be between 1:400 and 1:1, such as at least 1:300, e.g. at least 1:250, such as at least 1:200, e.g. at least 1:150, such as at least 1:100, e.g. at least 1:80; such as at least 1:70, e.g. at least 1:60, such as at least 1:50, e.g. at least 1:40. An example illustrating the described ratio is 0.2 mg plant material extracted with 3.5 mL of solvent and further diluted with 10 mL solvent.

The at least one standard colour scale may also be a description of the colours which can be the colours to determine i.e. possible colours of the extract and among which the user has to distinguish. The standard colour scale may comprise at least two colours and/or shades of colours, such as at least three colours and/or shades of colours, e.g. at least four colours and/or shades of colours, such as at least five colours and/or shades of colours, e.g. at least six colours and/or shades of colours, such as at least seven colours and/or shades of colours, e.g. at least eight colours and/or shades of colours, such as at least nine colours and/or shades of colours, e.g. at least ten colours and/or shades of colours, such as at least 11 colours and/or shades of colours, e.g. at least 12 colours and/or shades of colours, such as at least 13 colours and/or shades of colours, e.g. at least 14 colours and/or shades of colours, such as at least 15 colours and/or shades of colours, e.g. at least 16 colours and/or shades of colours, such as at least 17 colours and/or shades of colours, e.g. at least 18 colours and/or shades of colours, such as at least 19 colours and/or shades of colours, e.g. at least 20 colours and/or shades of colours.

The solvent and/or reagent described elsewhere herein may be substituted by antibodies to substances in one or more of the groups of compounds mentioned elsewhere herein. The antibodies when bound to phytochemicals may be detected by methods known in the art.

In an embodiment the kit comprises:
- at least one solid support (e.g. sticks and/or disks),
- at least one solvent,
- at least one squeezing means,
- optionally at least one standard colour scale,
- at least one glass/container.

The assay kit may further comprise one or more of the components selected from the group of:
- at least one chemical reagent,
- at least one mortar with pistil and/or at least one box with balls to shake and/or at least one hand-press
- at least one pipette,
- at least one UV-lamp,
- at least one heater and/or at least one warm cap made of chemical reagents and solvents,
- at least one balance,
- at least one scissor,
- at least one pair of forceps,
- at least one plastic bag,
- at least one identification information to identify plant species,
- at least one instruction describing how to use the assay kit,
- at least one syringe,
- at least one filter.

Examples of elements further included in the kit comprises one of the combinations, although any combination of the elements listed above is intended to be described:
- at least one chemical reagent, mortar with pistil and/or balls to shake and/or hand-press, pipette.
- at least one chemical reagent, heater and/or warm cap made of chemical reagents and solvents, identification information to identify plant species.
- at least one chemical reagent, mortar with pistil and/or balls to shake and/or hand-press, heater and/or warm cap made of chemical reagents and solvents, syringe, filter.
- heater and/or warm cap made of chemical reagents and solvents, balance, scissor, identification information to identify plant species, instruction describing how to use the assay kit.
- at least one chemical reagent, mortar with pistil and/or balls to shake and/or hand-press, pipette, heater and/or warm cap made of chemical reagents and solvents, scissor, pair of forceps, identification information to identify plant species, instruction describing how to use the assay kit, syringe, filter.

In a preferred embodiment the kit comprises the following components:
- 3 solid support (e.g. sticks and/or disks),
- 3 boxes with lids and each with 4 glass balls
- 6 glasses,
- 3 syringes each with 13.5 mL solvent,
- 1-3 containers with chemical reagents,
- 3 pipettes,
- 1 balance,
- 1 scissor,
- 1 pair of forceps,
- 3 plastic bags,
- 1 identification information to identify plant species,
- 1 instruction describing how to use the assay kit including a standard colour scale,
- 3 filters.

The components of the kit may be located in a box. The box may be of cardboard and/or plastic or any other suitable material.

The test kit may include components and reagents for performing three tests. In respect of a crop field treated with herbicide it is preferred that three samples of weed plants are collected three different places in the treated field. Each sample may be of e.g. 20-25 plants. The test kit may also include components for more than 3 tests, e.g. 4, 5, 6, 7, 8, 9, 10, 11, 12, 13, 14, 15, 16, 17, 18, 19, 20 or even more.

The glass balls of the kit may have any suitable size such as between 1 mM and 3 cM in diameter, e.g. between 1.1 mM and 1 cM, such as between 1.2 mM and 9 mM, e.g. between 1.3 mM and 8 mM, such as between 1.4 mM and 7 mM, e.g. between 1.5 mM and 6 mM, such as between 1.6 mM and 6 mM, e.g. between 1.7 mM and 5 mM, such as between 1.8 mM and 4 mM, e.g. between 1.9 mM and 3 mM, such as between 2 mM and 2.5 mM.

The glass balls are used to smash or squeeze the plant material by shaking a closed container containing the glass balls together with plant material and optionally a solvent.

The filter of the kit may be a filter paper used to filter the extract or it may be a filter cartridge including the filter, where the filter cartridge can be connected to a syringe. The syringe may be used to force the extract or reacted extract through the filter.

Containers with chemical reagents may have any suitable size, e.g. a size between 0.5 and 30 mL. The containers may include a volume of chemical reagent corresponding to a number of test to be performed e.g. to 2, 3, 4, 5, 6 or more tests.

Solvents and/or chemical reagents from a container or syringe may be used by dividing the volume such that the plant material is squeezed in a part of the solvent e.g. 3.5 mL e.g. by shaking for e.g. about 2 minutes and then again about ½ minute when e.g. 10 mL solvent is added.

In an embodiment the assay comprises components, where some are to be used several times and some components are disposable.

In one embodiment the components which can be re-used is squeezing means e.g. mortar with pistil and/or at least one box with balls to shake and/or at least one hand-press, standard colour scale, glass/containers, pipette, UV-lamp, heater, balance, scissor, pair of forceps, identification information to identify plant species, instruction describing how to use the assay kit, syringe, filter.

In another embodiment the components which can be re-used is squeezing means e.g. mortar with pistil and/or at least one box with balls to shake and/or at least one hand-press, standard colour scale, UV-lamp, heater, balance, scissor, pair of forceps, identification information to identify plant species, instruction describing how to use the assay kit.

In an embodiment the disposable components may be solid support (e.g. sticks and/or disks), solvent, squeezing means e.g. mortar with pistil and/or at least one box with balls to shake and/or at least one hand-press, standard colour scale, glasses, chemical reagent, pipette, UV-Iamp, heater and/or at least one warm cap made of chemical reagents and solvents, scissor, pair of forceps, plastic bag, identification information to identify plant species, instruction describing how to use the assay kit, syringe, filter.

In another embodiment the disposable components may be solid support (e.g. sticks and/or disks), solvent, squeezing means e.g. mortar with pistil and/or at least one box with balls to shake and/or at least one hand-press, glasses, chemical reagent, pipette, heater and/or at least one warm cap made of chemical reagents and solvents, plastic bag, syringe, filter.

The assay kit can be re-establish with disposable components after at least one use, hereby the kit is ready to use again.

It is a purpose of the invention to lower the costs and time of the testing procedure, and at the same time provide a method of testing having excellent sensitivity. The assay kit of the invention may for all practical purposes to be used as a field test, or as a laboratory test. One object of the invention is to have an easy accessible test to be used commercially or on a private scale. Thus, the assay kit of the invention is in one embodiment practical and portable in size and easy to operate. The test material is brought in contact with a support for receiving said material. The test material is in an assayable form, for example in the form of a liquid suspension.

In an embodiment the assay test kit is produced as a practical and mobile system which can be fully or partly disposable as described elsewhere. The individual step of the test may be performed in the field, and need not require any particular technical skills of the person performing the test. The test may be completed in less than 3 hours from the living organism is obtained, such as in less than 2 hours, e.g. less than 1 hour, such as less than 45 min, e.g. less than 30 min, such as less than 15 minutes.

In an embodiment the test method of the invention is performed with the following steps:
- Plant material (test material) is collected,
- The test material is cut into small pieces with the scissor and weighed on the balance,
- The material is brought into contact with a solvent,
- The mortar with a pistil, or a box with balls to shake, together with a solvent, crush out the sap of the plant material and/orA hand-press may be used to press out the sap of the plant material.
- The suspension is filtered through a filter.
- The extract Is brought into contact with chemical reagents
- After a chemical reaction is obtained the extract is contacted with a solid support e.g. a stick or disk.
- A colour will appear on the solid support,
- The colour on the solid support is compared to a standard colour scale, from this comparison the type of stress and/or the effect of the stress upon the plant can be determined and hereby it can be determined if the plant is resistant to the stress e.g. a pesticide or herbicide.

In an embodiment the test kit comprises containers with solvent and/or reagents, such as solvents and/or reagents described elsewhere herein. The containers may be in the form of e.g. flasks, glasses, and syringes. The containers may have a lid.

In another embodiment the assay kit comprises containers wherein the chemical reactions are performed. The(se) container may be containers with solvent and/or reagent.

In one embodiment all components of the assay kit are contained in one enclosure, this optionally being as a pocket size unit.

In one embodiment of the invention the assay kit is used on material from plants, and it may comprise:
- Thin Layer Chromatography (TLC) plates,
- eluents,
- reagents,
- hand-press,
- micro pipettes,
- optionally an UV-lamp,
- optionally a heater,
- at least one standard biomarker pattern,
- optionally at least one standard pattern.

In one embodiment the assay kit used on plant material comprises at least one TLCplate. The test material is brought into contact with a solid support, such as a TLC plate comprising an adsorbent material capable of separating the mixture of compounds of the test material. A hand-press may be used to press out the sap of the plant material. The assay kit also comprises containers of solvents, such as solvents described elsewhere. Containers, for example a bottle shaped container with a spraying head, holding reagents sprayed onto the TLC-plate(s) are also part of the present test kit. In another embodiment the assay kit comprises containers holding agents wherein the TLC-plates are placed. The containers may vary in size depending on the type of application in question. For repetitive tests larger containers may be preferred. In one aspect the assay kit is disposable, i.e. the individual components in the assay kit are used only once. However, in another aspect one or more of the individual components of the assay kit is recycled. Different embodiments of the assay kit of the invention are envisioned. In one embodiment all components of the assay kit are contained in one pocket size unit. In another embodiment the UV-lamp and/or the heater of the invention are separated from the assay kit as a unit. In another embodiment of the invention the UV-lamp and/or the heater are excluded from the assay kit and method as a whole. Further, micro-pipettes including holders are optionally included in the assay kit unit. The colour reactions developed on the solid supports, such as TLC-plates of the assay kit are compared to one or more colour comparators, such as the standard biomarker pattern and/or standard pattern of the invention. The colour comparators may be in the form of a folder of colour charts enclosed in the assay kit, and it is preferred that the size of the colour comparators (standard biomarker patterns and/or standard patterns) are equal to the size of the solid support, such as the TLC-plate(s).

In yet another aspect of the invention an immunological test, such as a "dipstick" is used.

As stated above, an aspect of the invention is the use of the method and kit for testing whether living organisms have developed resistance. In an embodiment the living organisms are plants.

In another embodiment the invention may be used by a farmer to determine the presence of resistance to pesticide e.g. herbicide.

The kit can be used to determine the presence of herbicide resistant weed biotypes prior or following a herbicide application.

In an embodiment the method or kit can be used for detecting pesticide resistance in weeds, pests and fungal diseases in arable crops.

The use of the method and/or assay as described herein may also be to test whether plants in a field are gene modified plants which are resistant to a specific herbicide.

The test kit including the standard colour scale may be designed to respond with a yes or no and optionally also a perhaps answer in respect of whether the plant is resistant to a predetermined herbicide.

The method of the tests described herein may also be designed to be based on a cut-off value by which a colour development indicates one answer e.g. resistant plants and where no colour development indicates another answer e.g. sensitive plants. This may especially be of value when performing a test to determine a "yes or no answer" as described elsewhere herein such as e.g. whether a plant is resistant to a herbicide. The colour indicating the value above or below a cut-off colour may be performed by the colour of the extract itself optionally reacted with one or more chemical reagents.

In another aspect the invention may be used in the control of the geographic distribution of resistance to pesticides. Non-target habitats adjacent to cultivated fields may be affected by pesticides during application. This exposure may occur due to over-spraying, or through spray drift from the application on target crops adjacent to wild-life habitats. It may also stem from pesticides being run-off or washed-off. Pesticides are able to travel considerable distances by air, either by drift or by volatilisation.

### Detailed description of Drawings

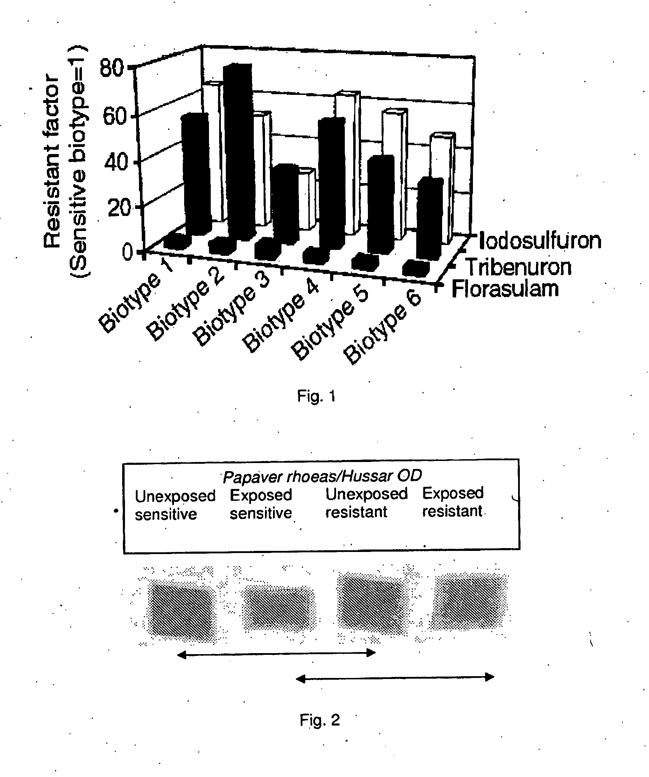

**Figure 1****.** Resistance factors of six different biotypes of the plant *Stellaria media* exposed to the herbicides active ingredients iodosulfuron, tribenuron and florasulam. The resistance factor is the ratio of the ED₅₀ value of the resistant and susceptible biotypes. If ED₅₀ of a susceptible biotype is 0.5 kg herbicide ha⁻¹ and the ED₅₀ of a resistant biotype is 20 kg herbicide ha⁻¹ then the resistance factor of the resistant biotype is 40. In figure 1 only the resistant biotypes are shown. The resistance factor of the susceptible biotype is 1.

### Experiments

### Example 1

### Herbicide resistance

In Denmark resistance to ALS inhibitors have been documented in *Stellaria media (11 locations)* and *Galeopsis sp. (1 location)* and a *Papaver rhoeas (7 locations)* biotype is being tested. Results from a study with 6 SU-resistant (Sulfonylurea-resistant) *Stellaria media* biotypes are included. The resistant factor of SU-resistant biotypes of *Stellaria media* to tribenuron and iodosulfuron varied from 27 to 77 (Fig. 1). A good correlation was found between the resistant levels to the two SU herbicides but cross-resistance to the ALS-inhibitor florasulam could not be confirmed.

Resistance to ACC-ase inhibitors has been documented in 17 *Alopecurus myosuroides* biotypes. Of the 17 biotypes 13 were shown to be target-site resistance, while 4 revealed a cross-resistance pattern typical for enhanced metabolism.

### Results

A biomarker pattern of 8 type A biomarkers were detected in extracts of unexposed *Stellaria media* plants. The same pattern was also detected in exposed plants 4, 7 and 14 days after exposure.

A biomarker pattern of 7 type B biomarkers were detected in extracts of susceptible plants 7 and 14 days after exposure. Five of these biomarkers were detected 4 days after exposure.

### Preliminary study

### Plants/Herbicide

A SU-susceptible and SU-resistant biotype of *Stellaria media* was cultivated outdoors in 2-litre pots. At the 4-5 leaf stage the plants were exposed to iodosulfuron (IOD) applied in mixture with a methylated vegetable oil (Renol). Iodosulfuron was applied at 50% of the recommended field dose. The plants were harvested 4, 7 and 14 days after exposure. Immediately after harvest the plants were frozen and freeze-dried.

### Sample preparation

Freeze-dried plant material (50 mg per ml of 75% ethanol) was extracted in ultrasonic bath with ice for two hours. The extracts were centrifuged before analysis.

### Biomarkers

A biomarker pattern was detected as phytochemical changes in plant extracts. A comparison of the extracts of an un-treated susceptible (Biotype 1) and an untreated resistant (Biotype 2) of *Stellaria media* is shown as biomarkers type A. See further description in Example 3.

A comparison of extracts of the same biotypes exposed to iodosulfuron is shown as biomarkers type B. See further description in Example 3.

High Performance Planar Chromatography (HPPC)-systems were used to detect the two types of biomarkers.

### Conclusions

A unique biomarker pattern could be detected in extracts of unexposed resistant *Stellaria media* (biotype 2) compared with susceptible (biotype 1) plants and this pattern did not change 4, 7 or 14 days after exposure to iodosulfuron.

A unique biomarker pattern was detected in extracts of the susceptible *Stellaria media* biotype 4, 7 and 14 days after exposure to iodosulfuron. This biomarker pattern was absent in the SU-resistant biotype.

### Example 2

### Development of a test-kit for determining sensitivity/resistance of plants in respect of herbicides

A stick/disk colour system to visualise the sensitivity of plants to herbicides (unexposed and exposed plants) compared with resistance (unexposed and exposed plants) are presented as a screening system. The differences in stick/disk colour are used to identify resistant plant species in the field either before exposure or after exposure to a herbicide. In conclusion, one colour is detected for sensitive plants which are unexposed to the herbicide, another colour for the sensitive plants exposed to herbicide and yet another colour is obtained if the plants are resistant (target or metabolic) and exposed to herbicides. The sensitive and resistant biotypes of the plant species showed a stable colour pattern when the plant material was analysed. Determination of sensitivity or resistance can be made e.g. seven to fourteen days after the plant is exposed to herbicide.

### Semi-field study

Sensitive and resistant biotypes of certain plant species were grown in pots outdoors (semi-field). Following herbicide treatment a connection between the stick/disk-colour and sensibility/resistance of the plants could be detected 14 days after the exposure.

### The reaction of plants to herbicide stress

Generally plants react to stress. In the development of the method used in the present invention it was observed that plant species sensitive or resistant to specific herbicides react differently to the stress performed as e.g. exposure to herbicides. The reaction to the stress can be observed as a biochemical responses. Herbicides are developed to affect general or specific mechanisms in plants with the result that sensitive plants will be disturbed in the growth and eventually the plant may die. When the normal mechanisms in the plants are disturbed, naturally the plant will react changing the concentration of already present compounds, produce new compounds or stop the production of compounds. The specific changes depend of the mode of action of the herbicide and the used dose.

A plant has a natural content of phytochemical compounds. The content of the phytochemical compounds varies in different plant species and the composition is different if the plants are sensitive or resistant to herbicides. The sensitive and the resistant plants react differently to the herbicide stress and hereby when performing a test according to the invention, a different colur will be present for the chemical reaction with the plant extract. The different colours can be observed even though the tested plant species are parts of the same family. When performing a herbicide treatment of the plants, the phytochemical composition and concentration of this composition changes in the plants. It has turned out that these changes can be detected in a very simple way. The changes in the phytochemical composition and concentration in the plants after herbicides stress exposure is called biomarkers. The biomarker method builds on a pattern of biomarkers developed in plants exposed to herbicides.

Preliminary investigations performed in green-house and in the field, underline the method. Significant changes in the phytochemical composition and content in wild mono- and dicotyledon plants could be detected as early as four days after exposure to herbicides down to 1% of recommended field-dose of the herbicides. The changes in biomarkers can be detected much earlier than the visual signs appear. In plants exposed to glyphosate (Tradename Roundup Bio), phytochemical changes in composition and content (biomarker pattern) could be detected four hours after herbicide exposure.

Further investigations have been performed on 16 different wild plant species (both mono- and dicotyledons) and a clear pattern of biomarkers was detected for four different herbicides with four different mode of action. The results showed, the higher concentration of the herbicides, the shorter time after the exposure the biomarkers could be detected.

Preliminary investigations with young plants and older plants showed that young plants reacted faster after herbicide exposure than older plants.

The present invention consists of a method that utilises the different phytochemical composition before and after herbicide exposure to identify resistant plants directly in the field. The phytochemical composition is visualised by a colour reaction in a kit. The examples show that the kit can be used to test if a plant is sensitive (tested on unexposed and exposed plants) or resistant (if definitive response on exposed plants and probably identification also of untreated resistant plants).

### Plants

The plant species used for the studies are all common weed plants in crops in Denmark. The plant species were: Silky-bent grass (*Apera spica-venti* L., Beauv), foxtail grass (*Alopecurus myosuroides* Hudson), chickweed (*Stellaria media* L. (Will.) and common red poppy (*Papaver rhoeas* L.).

### Herbicides

The herbicides tested were: Hussar OD (iodosutfuron + mefenpyr-diethyl, (100 + 300) g/l, Bayer CropScience A/S); Atlantis WG (mesosulfuron + iodosulfuron + mefenpyr diethyl, (30 + 6 + 90) g/kg, Bayer CropScience A/S); Primera Super (fenoxaprop + 0.2% isoblette or 0.1% Contact) Bayer CropScience A/S). Table 1 presents an overview of plant species, biotypes, herbicides and treatments of the studies.

**Table 1: Overview of studies, plant species, biotypes, herbicides and treatments.**

| **Code** | **Plant species, stages and biotypes** | **Herbicide** | **Treatment** | **Fresh/frozen/ Freeze-dried** |
|---|---|---|---|---|
| 952/06 | *Apera spica-venti* (5-6 leaves, 2-3 offshoots) 5 biotypes (2 sensitive, 3 target resistant) | Atlantis 1N = 20 g a.i./ha (+ 0.5 L/ha Renol) | Semi-field | Fresh frozen |
| 905/07 | *Stellaria media* (6 leaves), 3 biotypes (1 sensitive, 2 target resistant) | Hussar OD 1N = 0.07 g a.i./ha (+ 0.5 L/ha Renol) | Semi-field | Fresh frozen |
| 905/07 | *Papaver rhoeas* (6-8 leaves) 4 biotypes(1 sensitive, 3 resistant) | Hussar OD 1N =0.2 g a.i./ha (+0.5 L/Ha Renol) | Semi-field | Fresh frozen |
| 905/07 | *Alopecurus myosuroides* (4 leaves), 3 biotypes (1 sensitive, 1 target, metabolic resistant) | Primera Super 1N = 0.2 L/Ha =13.8 g a.i./ha | Semi-field | Fresh frozen |
| 941/07 | *Apera spica-venti* (4 leaves, 1 offshoots) 7 biotypes (2 sensitive, 5 target resistant) | Hussar OD 1N = 24 g a.i./ha | Semi-field | Fresh frozen |

### Semi-field study - Cultivation and exposure of plant species

The weed species were sown in 2 L pots in a mixture of field soil, sand and sphagnum (2:1:1 weight per cent). After sowing, the pots were placed on out-door tables, where they were watered several times per day. After emergence the plants in the pots were thinned to the same number of plants in all the pots. The herbicides were applied when the plants were at the defined growth stage (se table 1). The herbicides were applied in deionised water using a laboratory pot sprayer fitted with two ISO F-02-110 flat fan nozzles delivering a spray volume of 145 L per Ha with a pressure on 3 bar.

The normal dose (1N) used varies between the studies depending of the sensitivity of the weed species and the growth stage of the plants. All the biotypes of the plants were exposed to 1 N dose (see table 1) and for the target resistant plants, 8N (941/07 and 941/07) and 10 N (952/06) was also used to confirm the resistance in the different biotypes. Since the purpose of the studies primarily was to identify herbicide resistance in plants, the colour reaction on the stick of the sensitive and the herbicide resistant biotypes were compared for treatments with 0N (untreated) and normal dose (1 N).

In the experiments the plants used for the biomarker test were harvested 14 days after exposure with the herbicides. The plants were cut at the surface of the soil and frozen immediately by the use of dry-ice. Before harvest a visual evaluation of effect (morphological changes) was performed, this is further described below. The used scale for evaluation of the morphological changes is described in table 2. Except for study 941/07, biomass determinations were performed by harvesting plant biomass 21 days after herbicide application, as described below. Before the harvest of biomass, a visual evaluation of the effect was performed.

### Visual effect evaluation

The visual effect on the plants before sampling was assessed using the scale shown in table 2 where 0 is no effect on the plants and 9 indicate dead plants (Hamil *et al.,* 1977; Boutin *et al.,* 1993).

**Table 2: Visual effect evaluation. The scale is used to evaluate the visual effects of plants exposed to herbicides.**

| | |
|---|---|
| **VE: V**isual **E**ffect on plants exposed to herbicides (0-9): | |

| **Skala** | **Detailed description** |
|---|---|
| 0 | **No effect** |
| 1 | **Trace effect:** Normal look with a slight stimulated growth |
| 2 | **Weak effect** |
| 3 | **Moderate effect:** plants 75% of size of untreated plants (reduced with 25%) |
| 4 | **Damage:** plants more than 50% of the size of untreated plants and with marked injuries on leaves and stems |
| 5 | **Injury damage:** plants half size of untreated plants, curved leaves, plant parts deformed and miscoloured |
| 6 | **Herbicide effect:** plants 25% of the size of untreated plants, curved leaves, plant parts deformed and miscoloured |
| 7 | **Good herbicide effect:** very small plants, curved leaves, plant parts deformed and miscoloured |
| 8 | **Nearly dead:** only few green plant parts left |
| 9 | **Dead** |

### Biomass evaluation

In the semi-field studies the biomass determination was performed 21 days after herbicide application. The fresh weight was calculated as mean of 3 replicates for each treatment The dry weight was recorded after the plant material was air dried at 80° C for 18 hours.

### Techniques used for method development

Thin Layer Chromatography (TLC) was used to separate and select relevant biomarkers and biomarker groups (see reports Appendix 1).

### Collection of weed plants

Plants from each pot (three replicates) were collected 14 days after exposure with the herbicide. The plants were immediately frozen with dry-ice and kept frozen at-18°C until analysis.

### Colour

Colour and colour intensity of sticks from test of plants from semi-field studies were compared with a PANTONE^{®}formula guide uncoated scale and also analysed using advanced CAMAG picture analytical equipment for documentation (see Appendix 2).

The colours referred to as PANTONE-colours are as in PANTONE Formula Guide/ Solid Uncoated ISBN 978-1-590650-63-9 Fourth Edition Second Printing.

### Preparation of plant extract

Fresh or frozen plant material was cut into small pieces with a scissor. 2.00 g/1.00g fresh plant material was weighed on a KERN 60-2N Pocket balance (max = 60g d = 0.01g) and crushed in a mortar (inner diameter = 5.2 cm) with 40.00 ml deionised water. The extract was filtered through a WHATMAN 0.45 µm GMF w/GMF filter. This filtered extract was used for screening

### Screening method

Plant extracts (both 50 mg/ml and 25 mg/ml) of the sensitive (unexposed and exposed) plants were screened in the screening programme. The results were evaluated and the concentration of the extracts was chosen for the resistant plants. The most important factor for the choise of extracts was a distinct difference in colour of extracts of the exposed plants compared with extracts of the unexposed plants. The concentration of the extracts turned out to be an important factor.

### Results and discussion

The development of the test-kit was performed in four phases.
Phase 1: Screening of the plant extracts in the new screening system; Phase 2: Selection of the system and further method development; Phase 3: Development of test-kit; Phase 4: Validation of test-kit.

First, in phase 1, the comparison between the colours of the chemical test for extracts obtained from the unexposed and the exposed plant of a sensitive biotype was performed. The test method and the amount of extract to be tested were selected such that a clear difference in the colour reaction was present for the comparison. Hereby it is possible to evaluate if the reaction is based on herbicide sensitive plants, which has not been sprayed, instead of biotypes with resistance to the herbicides after spraying. The next comparison is a clear difference in colour between the sensitive, exposed plant biotypes and the resistant, exposed biotypes. Hereby, the herbicide resistant plants can be detected. Finally, if the differences in colours are detected between the sensitive, unexposed and exposed plants, and herbicides resistant plants which were unexposed and exposed, a test-kit can be developed to be used before and after exposure to herbicides in the field. It is acceptable that herbicide resistance in the plants can only be identified after exposure, while the differences in colours of unexposed and exposed sensitive plants and exposed resistant plants are required.

An overview of the different plant biotypes and herbicides are presented in table 3.

**Table 3. Plant species, biotypes and herbicides used in the development of the method and test kit. ID numbers refer to identification numbers in the seed bank at Dept. of Integrated Pest Management. Code refer to trial number.**

| *Monocotyledons* | | | | **Code** |
|---|---|---|---|---|
| ***Apera spica-venti*/Atlantis** | | | | **DJF 952/06** |
| Biotype 1 | Biotype 2 | Biotype 4 | Biotype 5 | Biotype 3 |
| ID 100 | ID 452 | ID 454 | ID 455 | ID 453 |
| Sensitive | Sensitive | Sensitive | Metabolic resistant | Target resistant |
| | | | | |
| | | | | |
| ***Alopecurus myosuroides* /Primera Super + Isoblette** | | | | **DJF 905/07** |
| Biotype 7 | Biotype 8 | Biotype 9 | | |
| ID 85 | ID 31 | ID 19 | | |
| Sensitive | Target resistant | Metabolic resistant | | |
| | | | | |
| *Dicotyledons* | | | | |
| ***Stellaria media* /Hussar OD + Renol** | | | | **DJF 905/07** |
| Biotype 1 | Biotype 2 | Biotype 3 | | |
| ID 1 | ID 9 | ID 102 | | |
| Sensitive | Target resistant | Target resistant | | |
| ***Papaver rhoeas* /Hussar OD + Renol** | | | | **DJF 905/07** |
| Biotype 4 | Biotype 5 | Biotype 6 | Biotype 12 | |
| ID 150 | ID397 | ID 248 | ID 406 | |
| Sensitive | Target resistant | Target resistant | Target resistant | |

In table 4 this comparison is presented for the screening system for combinations of biotypes of the four plant species and herbicides. The numbers in the table indicates the different colour. See Appendix 3 for the reagent codes.

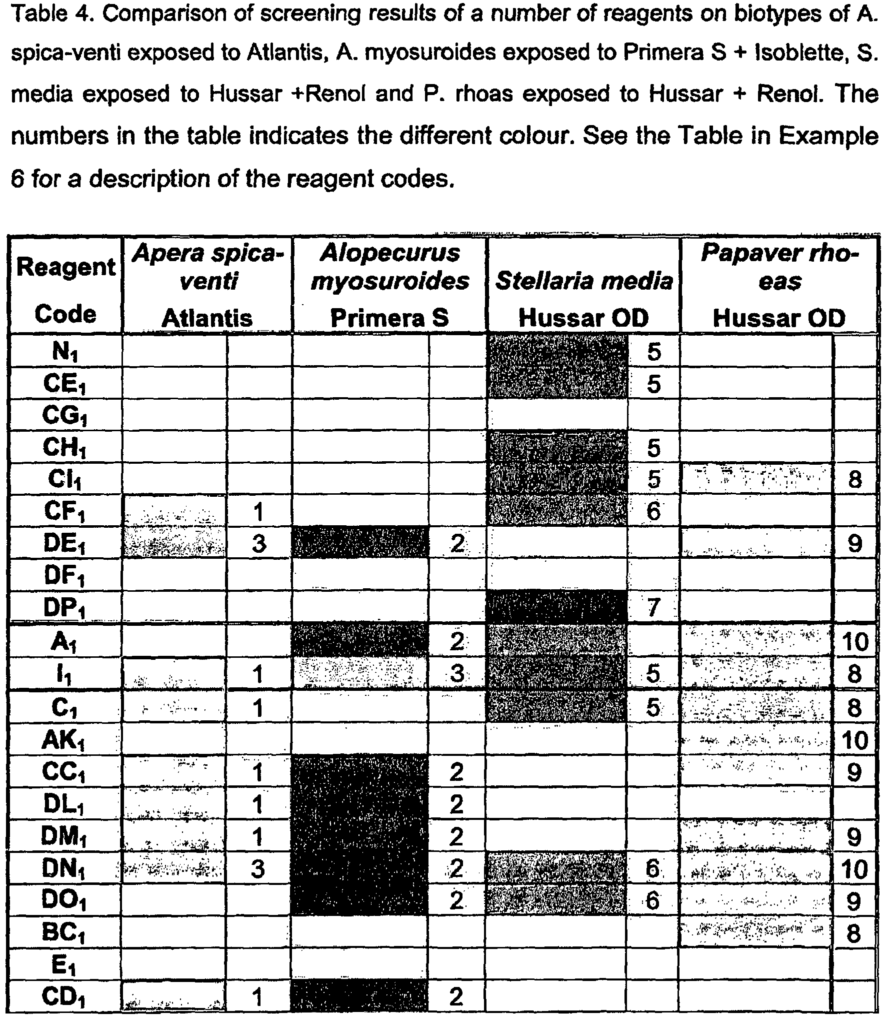

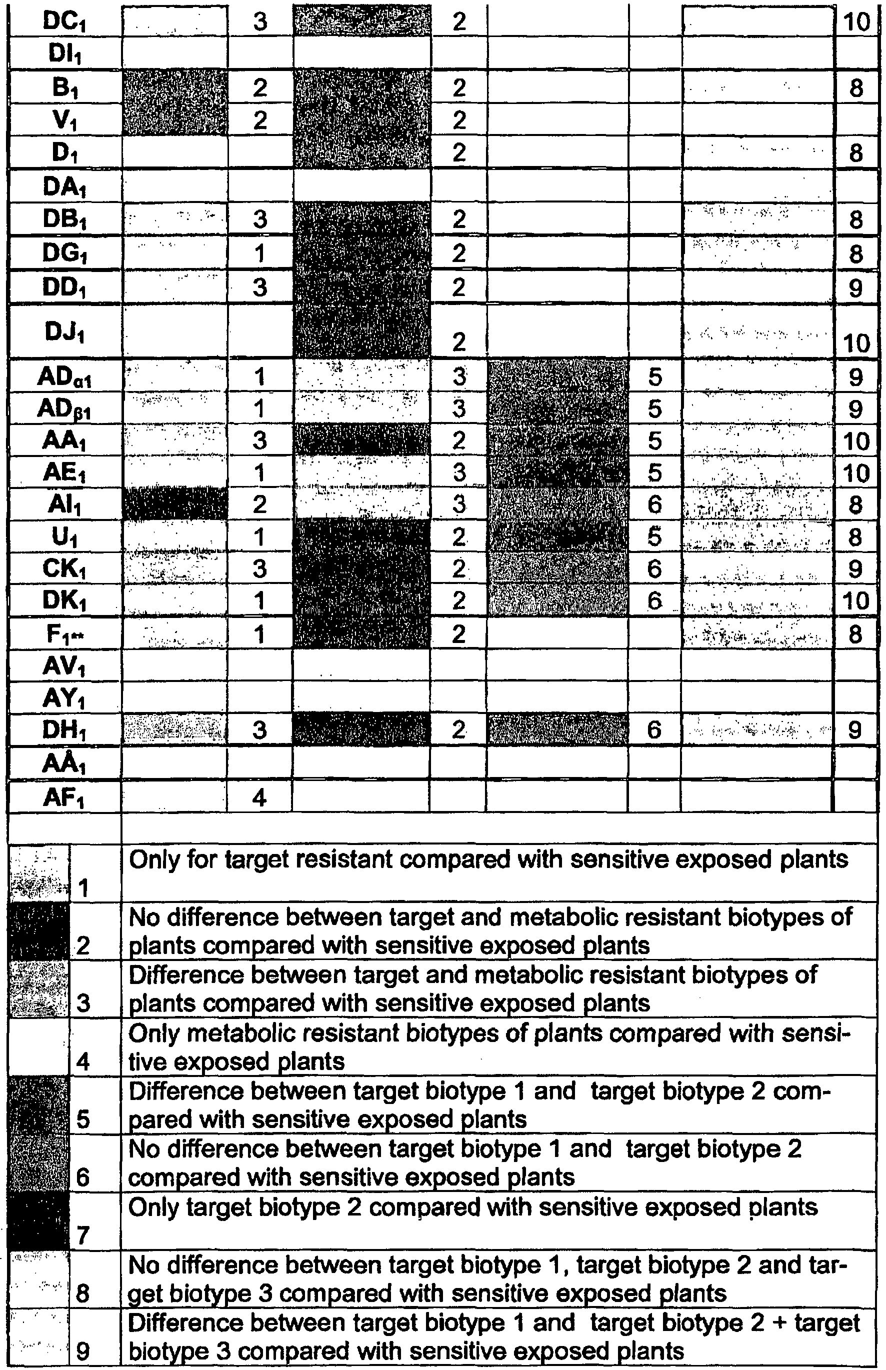

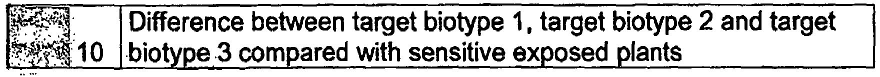

Table 5 summarizes responses for all four plants species to the most potential reagents

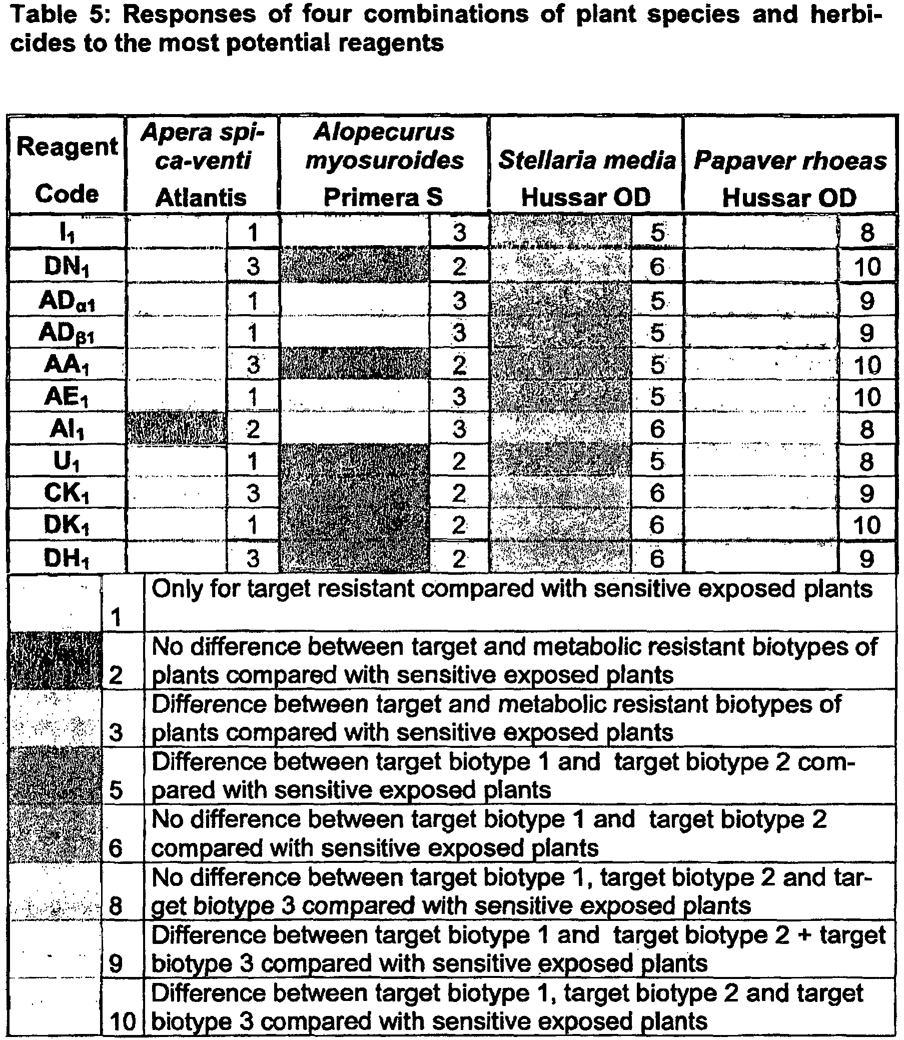

Since none of the reagents seemed to be optimal for development of a common system for all plant species and herbicides it was decided to make a priority list for development of methods: 1) resistance in *Papaver rhoeas;* 2) resistance in *Apera spica-venti:* 3) resistance in *Stellaria media,* all with sulfonylurea resistance.

It was further decided to continue with the reagents with the codes: I₁, A₁, AD_{α1}, AD_{β1}, AD_{β1}, AE₁, AI₁, DG₁, and DM₁.

Different combinations of these reagents were mixed and investigated to be included in a test-kit for identifying of herbicide resistance in plants.

### An example:

A mixture of I₁, A₁ AD_{α1} (1:1:1) 4 drops + 10 drops of plant extract (50 mg/ml extracted with water) + 15 drops of conc. sulphuric acid present the colour picture of Fig. 2.

Optimal colour difference having four different colours. The colours are (from left to right) PANTONE^{®} no. 535 (blue-gray); no. 452 (green-gray); no. 7544 (gray), no. 415 (gray-green). The final test may include two steps. Step 1: The result is a resistant plant. To confirm that the plant is resistant and not just an unexposed plant step 2 is performed. Step 1 and step 2 include different combination of reagents.

### References

Boutin, C.; Freemark, K.E. & Keddy, C.J. (1993): Proposed guidelines for registration of chemical pesticides: Nontarget plant testing and evaluation. Technical Report Series. No. 145. 1-91. Ottawa. Canadian Wildlife Service. Environment Canada.

Hamil, A.I.; Marriage, P.B. & Friesen, G. (1977): A method for assessing herbicide performance in small plot experiments. Weed Sciences. 25. 386-389.

Snyder, L.R (1974) Journal of Chromatography A, 92, 2, 233-230.

### Example 3

### Thin Layer Chromatography (TLC) analysis of Papaver rhoeas (Papaveraceae)

### Plant, biotypes and extracts

S₀ = ID 150 Biotype 4 sensitive control
S₁ = ID 150 Biotype 4 sensitive exposed to 1N Hussar OD+0.5 l/ha Renol
R₀ = ID 397 Biotype 5 Target resistant control
R₁ = ID 397 Biotype 5 Target resistant exposed to 1 N Hussar OD+0.5 l/ha Renol
R₁₀ = ID 397 Biotype 5 Target resistant exposed to 10N Hussar OD+0.5 l/ha Renol
R₂₀ = ID 248 Biotype 6 Target resistant exposed control
R₂₁ = ID 248 Biotype 6 Target resistant exposed to 1N Hussar OD+₀.₅ l/ha Renol
R₂₁₀= ID 248 Biotype 6 Target resistant exposed to 10N Hussar OD+0.5 l/ha Renol
R₃₀ = ID 406 Biotype 12 Target resistant exposed control
R₃₁ = ID 406 Biotype 12 Target resistant exposed to 1N Hussar OD+0.5 l/ha Renol
R₃₁₀= ID 406 Biotype 12 Target resistant exposed to 10N Hussar OD+0.5 l/ha Renol

### Extracts

100 mg freeze-dried plant material (*Papaver rhoeas* (Papaveraceae) in 4.00 ml 80% Ethanol extracted for 20 min. in ultrasonic bath (Realisation Etudes Ultra Son (RE-US), 16, allée des Cystes, 06390 Contes, France). The extract was filtered through GELMAN GHP Agrodisc 13 0.45 µm.

### Thin Layer Chromatography (TLC)

The extracts (20µl) were applicated on Silicagel 60 Merck 1.05721 (10x20 cm) or (10µl) Cellulose aluminium Merck 1.05552.0001 together with the refererence 10/00 papaverin in ethanol using a TLC Automatic TLC Sampler III.

| S₀ R₀ R₂₀ R₃₀ | S₁ R₁ R₂₁ R₃₁ | R₁₀ R₂₁₀ R₃₁₀ |
|---|---|---|
| Control | Exposed 1N | Exposed 10N |

### Results

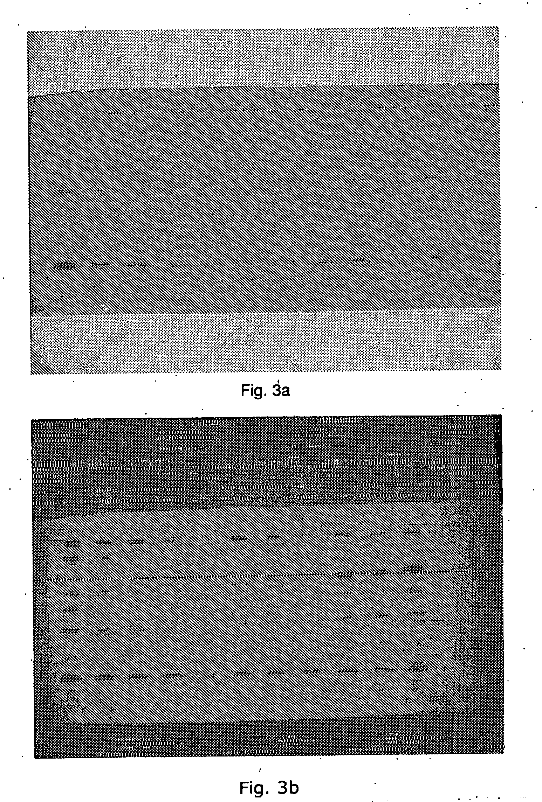

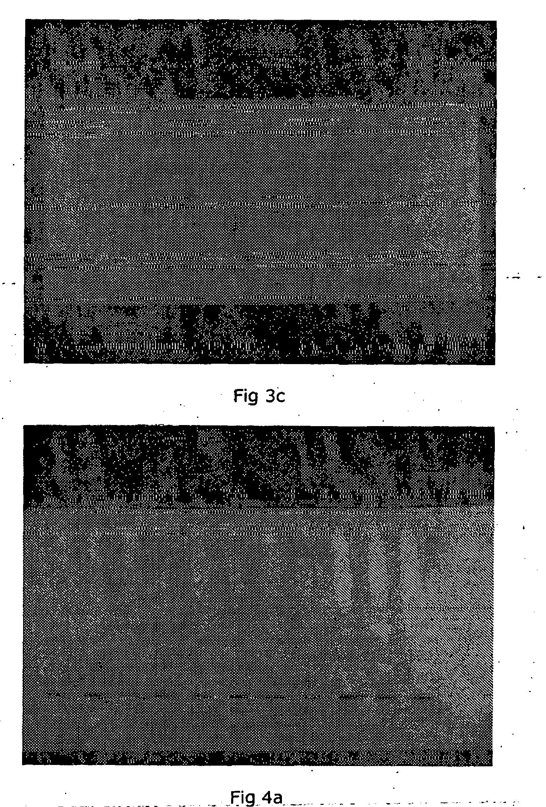

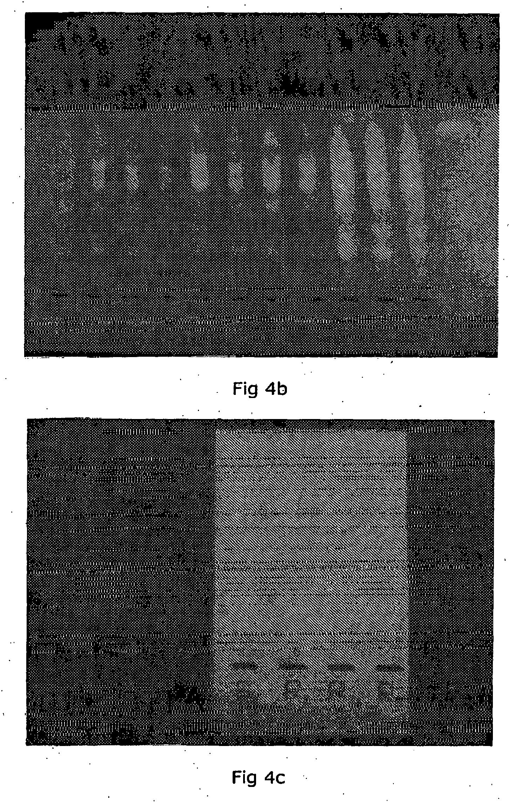

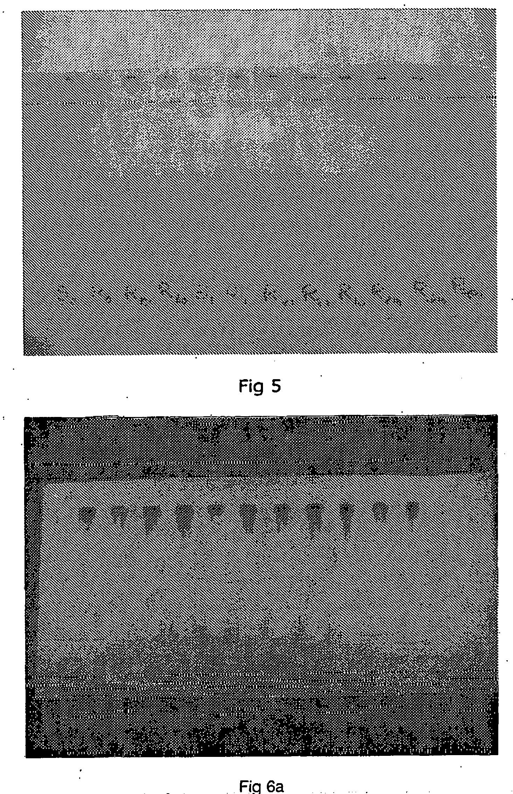

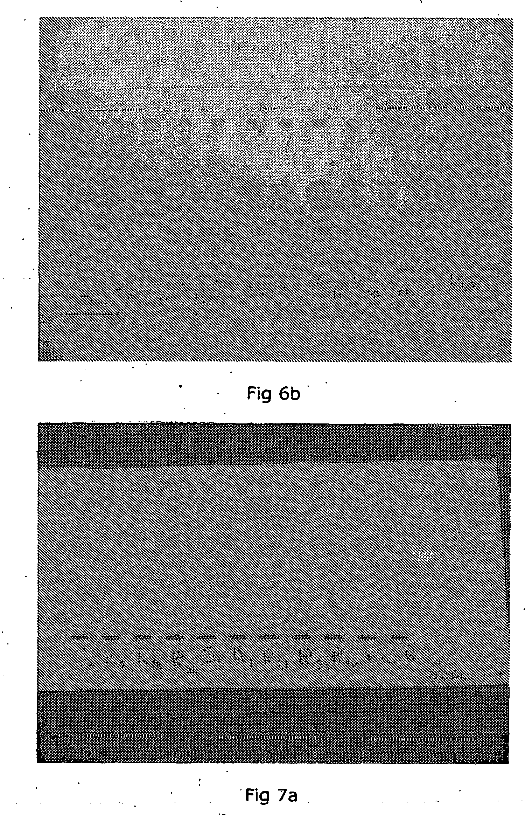

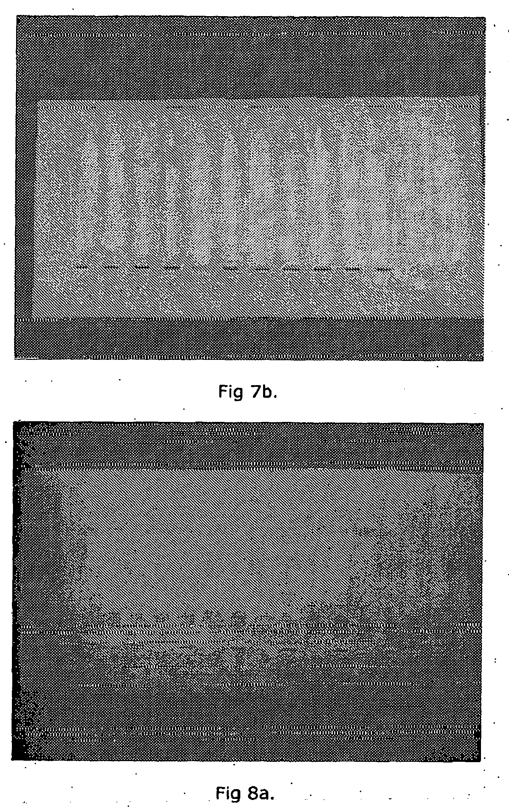

TLC 1): Plate: Silica gel 60; Solvent: Toluene: Ethyl acetate: Diethylamine (70:20:10). The results are shown in Fig. 3.
   Visual detection is seen in Fig 3a.
   UV 366 nm Detection is seen in Fig 3b.
   UV 366 nm detection after derivatisation with: 1% NEU and 5% PEG is seen in Fig 3c.
TLC 2): Plate: Cellulose; Solvent: 15% Acetic acid in water. The results are shown in Fig. 4.
   UV 366 nm detection is seen in Fig. 4a.
   UV 366 nm detection after treatment with 1% 2-amino diphenylborinate in methanol (NEU) and 5% polyethyleneglycol 4000 in 50% ethanol (PEG) is seen in Fig 4b and 4c.
TLC 3) Plate: Silica gel 60; Solvent: n-butanol : Methanol : Water (30:30:40). The results can be seen in Fig 5.
   Visual detection after derivatisation with Dragendorff* (Merck no. 197) is seen in Fig 5.
      * Dragendorff (Merck no 197) = Main solvent: 14 g IK + 6 ml 4N HCl + 36 ml water, after heating 3 g Bismuth nitrate is added, after cooling 3 g I added. Diluted 1:1 with water. Solvent used: 2 ml main solvent + 8 ml 25% HCl.
TLC 4) Plate: Cellulose; Solvent: Isopropanol: Acetic acid: Water (70:5:25). The results can be seen in Fig 6.
   Detection: UV 366 nm before derivatisation is seen in Fig 6a.
   After derivatisation with ninhydrin** (Merck no 179) is seen in Fig 6b.
      **Ninhydrin (Merck no. 179) = I: Ninhydrin 100 mg + 10 ml Acetic acid + 2 ml Collidine + Cadmium acetate 250 mg in 50 ml ethanol (absolute); II: Copper nitrate = 1% in absolute ethanol. I + II : 10 ml + 0.6 ml. After spray heat for 3 minutes at 100°C.
TLC 5) Plate: Cellulose; Solvent: 2% Acetic acid in Water. The results can be seen in Fig 7.
   Detection: UV 366 nm before derivatisation is seen in Fig 7a.
   Detection: UV 366 nm after derivatisation with NEU + PEG is seen in Fig 7b.

### Evaluation

### TLC 1:

The TLC system is very efficient for chlorophyll and carotenoids separation and detection. Differences were detected between biotypes of the sensitive and the target resistant plants. Compounds were detected in the biotypes of the resistant plants and not detected in the sensitive plants. However, the best results were seen for the exposed plants. A tendency of sensitive exposed plant is similar with target resistant exposed to 10N Hussar OD + Renol. This also concerns the phenolic compounds at the baseline (UV detection at 366 nm).

### TLC2:

The TLC system after derivatisation with NEU and PEG was the best. A difference in content of flavonoids and phenolic compounds were detected between the biotypes of the sensitive and the target resistant plants unexposed and exposed. However, biotype of the sensitive exposed seems to have the same content as for the 10 N exposed target resistant plants. The blue-white phenolic compounds at Rf = 0.36 is only present in the sensitive plant and the target resistant plants exposed to 10N.

Also the blue-white phenolic compounds at Rf =0,61 and 0.64 is present in a higher amount in the biotypes of target resistant exposed plants compared with the sensitive plants. The flavonid at Rf = 0.27 is present in all biotypes but in less amount in the unexposed sensitive and the target resistant biotype no. 406.

### TLC 3:

This analysis was performed since an interesting reaction at histochemical investigations was detected for the same reagent.

Alkaloids were detected in all biotypes at Rf = 0.88. The reference papaverin have the same Rf value, but the colour is different from papaverin which is dark orange with Dragendorff reagent. Papaverin is slight yellow in UV before devivatisation and not detected with ninhydrin reagent.

### TLC 4:

Concerning the amino acids, the composition of the sensitive unexposed was different from the target resistant plants unexposed and exposed. However, the sensitive unexposed plants compared with the exposed sensitive plants are very different.

The sensitive exposed plants seem to have the same composition as the target resistant plants exposed with 10N. This was also seen for the phenolic compounds.

Two compounds at Rf = 0.12 and Rf = 0.52 are only present in the target resistant biotypes and in the sensitive exposed biotype. An identification of the amino acids would be interesting for further development of this aspect.

### TLC 5:

A very remarkable flavonoid (yellow spot) is detected for the sensitive plant exposed to iodosulfuron. The flavonoid is placed as shown for the top of the arrow. The compounds as verbascoside (V) yellow in not present. However, the chlorogenic-type compounds are present in the plants in high amount in the sensitive exposed biotype and the exposed target biotypes with 10N (as seen in other TLC systems).

### Conclusion

The content of phenolic compounds as simple phenolic compounds and flavonoids are interesting for further development of the project, since a new flavonoid in the sensitive plant exposed to iodosulfuron is present. This compound is not present in the biotypes of the target resistant plants.

The composition of amino acids may also be important for the identification of target resistant unexposed and exposed plants.

### Example 4

### Thin Layer Chromatography (TLC) analysis of Stellaria media (Caryophyllaceae)

### Plant, biotypes and extracts

S₀ = ID 1 Biotype 1 sensitive control
S₁ = ID 1 Biotype 1 sensitive exposed to 1N Hussar OD+0.5 l/ha Renol
R₁₀ = ID 9 Biotype 2 Target resistant control
R₁₁ = ID 9 Biotype 2 Target resistant exposed to 1N Hussar OD+0.5 l/ha Renol
R₁₁₀ = ID 9 Biotype 2 Target resistant exposed to 10N Hussar OD+0.5 l/ha Renol
R₂₀ = ID 102 Biotype 3 Target resistant exposed control
R₂₁ = ID 102 Biotype 3 Target resistant exposed to 1 N Hussar OD+0.5 l/ha Renol
R₂₁₀= ID 102 Biotype 3 Target resistant exposed to 10N Hussar OD+0.5 l/lha Renol

### Extracts

Et = 100 mg freeze-dried plant material in 4.00 ml 80% Ethanol extracted for 20 min. in ultrasonic bath (Réalisation Etudes Ultra Son (REUS), 16, allée des Cystes, 06390 Contes, France).

Aq = 50 mg freeze-dried plant material in 4.00 ml 80% Ethanol extracted for 20 min. in ultrasonic bath (Réalisation Etudes Ultra Son (REUS), 16, allée des Cystes, 06390 Contes, France) + 200 µl EtOH after filtretion.

The extract was filtered through GELMAN GHP Agrodisc 13 0.45 µm.

### Thin Layer Chromatography (TLC)

The extracts (10µl) were applicated on Silicagel 60 Merck 1.05721 (10x20 cm) or (5µl) Cellulose aluminium Merck 1.05552.0001 with together with the refererence T = 1 0/00 X in ethanol using a TLC Automatic TLC Sampler III.

Position on TLC-plates:
0-T = References
1-S_{0Aq}
2-S_{0Et}
3-S_{1Aq}
4-S_{1Et}

5-R_{10Aq}
6-R_{10Et}
7-R_{11Aq}
8-R_{11Et}
9-R_{110Aq}
10-R_{110Et}

11-R_{20Aq}
12-R_{20Et}
13-R_{21Aq}
14-R_{21Et}
15-R_{210Aq}
16-R_{210Et}

### Solvents (eluents)ltype of TLC-plates:

S1 = 2% Acetic acid in water/cellulose
S2 = Dichloromethane:Acetic acid:Water (50:45:15)/cellulose
S3 = 2-propanol:Acetic acid:water (70:5:25)/cellulose
S4 = Ethylacetate:Formic acid:Acetic acid:water(100:11:11:25)
S5 = 2-propanol:Ethylacetate:water (7:2:1)/Silica
S6 = 15% Acetic acidin water /cellulose

### Derivatisation reagents:

A = Neu/PEG =1% 2-aminoethyl diphenylborinate in MeOH, the plate is dried and sprayed with polyethyleneglycol 4000 in 50% EtOH in water
B = Ninhydrine = 8% ninhydrine in EtOH. Heat 100°C for 3-5min.
C = p-anisaldehyde =100µl is mixed with 2.00 ml acetic acid, 17 ml MeOH and 1.00 ml sulphuric acid. Heat 100°C for 3-5min.
D = TTZ = 4% Triphenyltetrazolium in MeOH:1N NaOH in water (. Heat 100°C for 3-5 min. Colouration of simple sugars.
E = Naphthoresorcinol =0,2g naphthoresorcinol in 95.00 ml Abs. EtOH. 8ml of this solution + 2.00 ml phosphoric acid. Heat 100°C for 3-5min.
F = Benedict (Flavonoids type: apigenine) = 1.73g CuSO₄(5H₂O) + 17,3g sodium citrate + 10gCO₃Na₂ in 200 ml water.

### Results

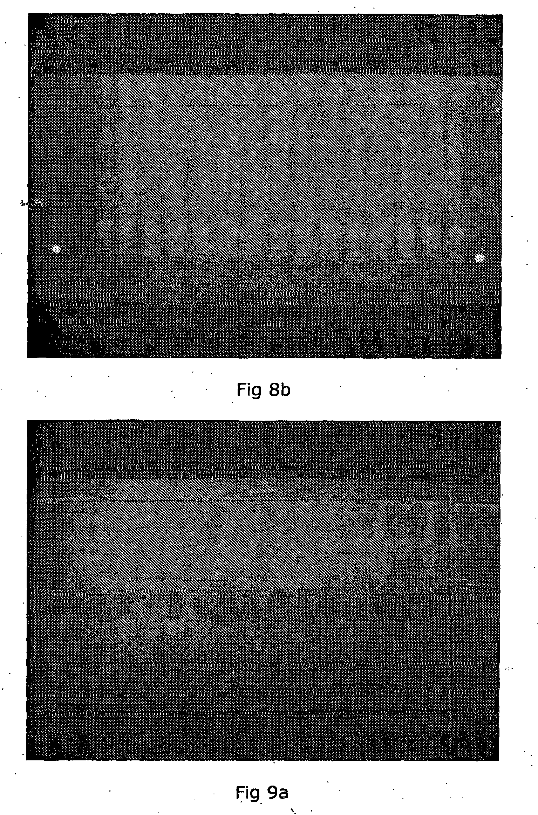

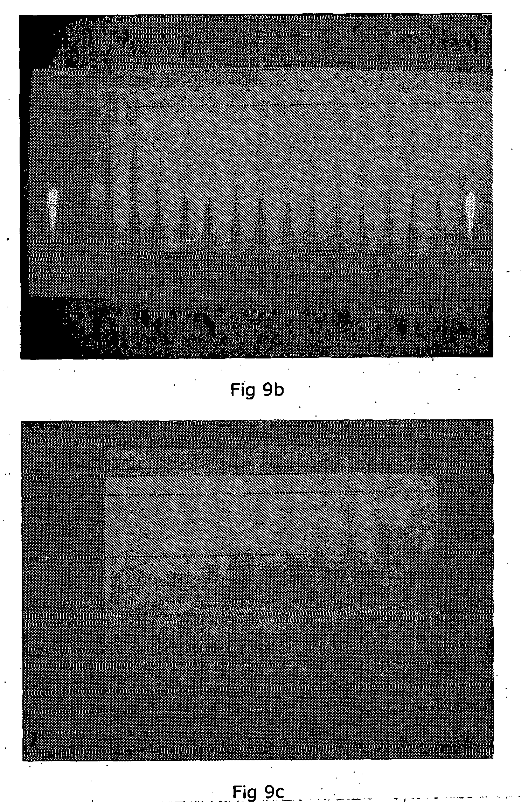

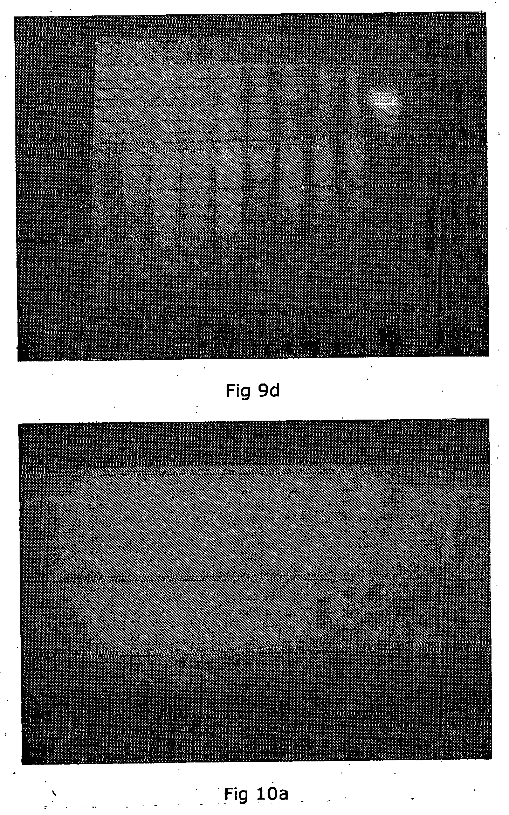

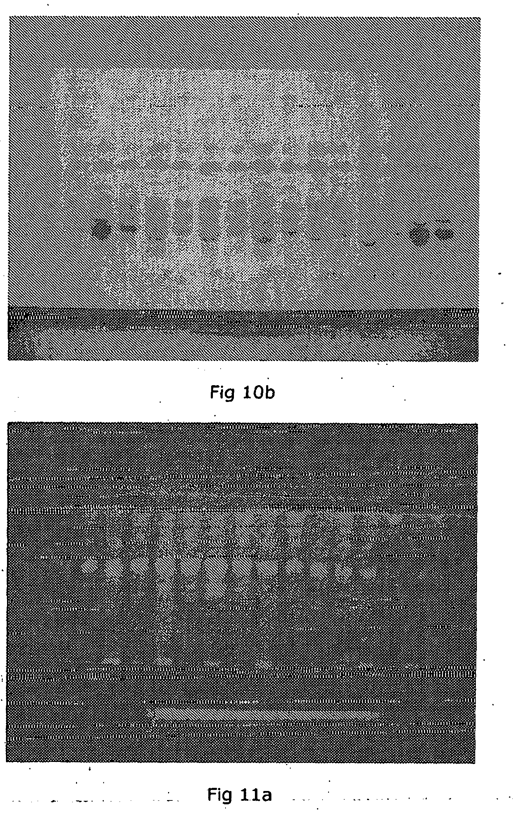

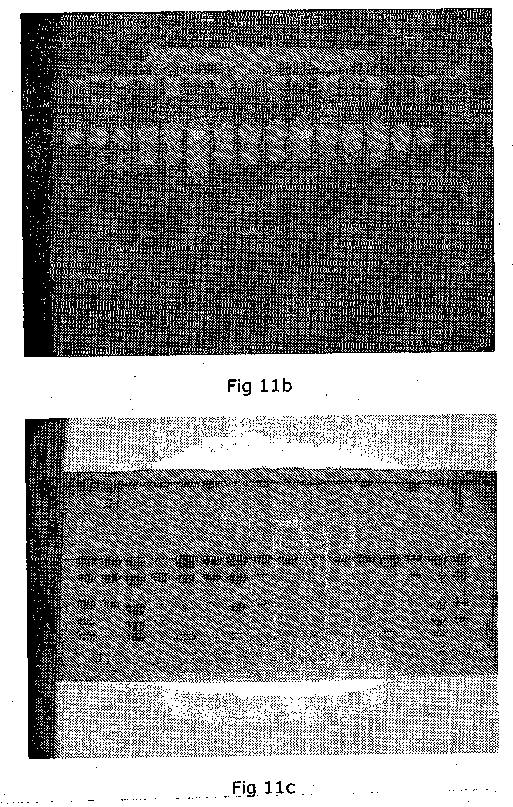

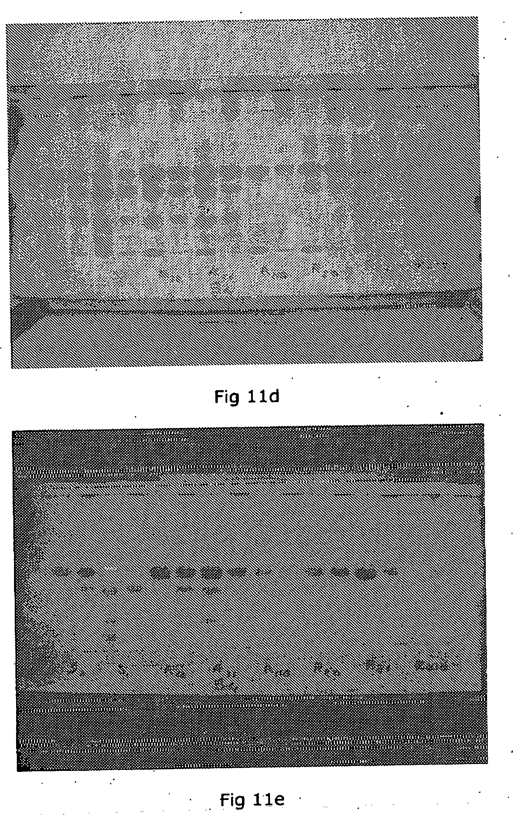

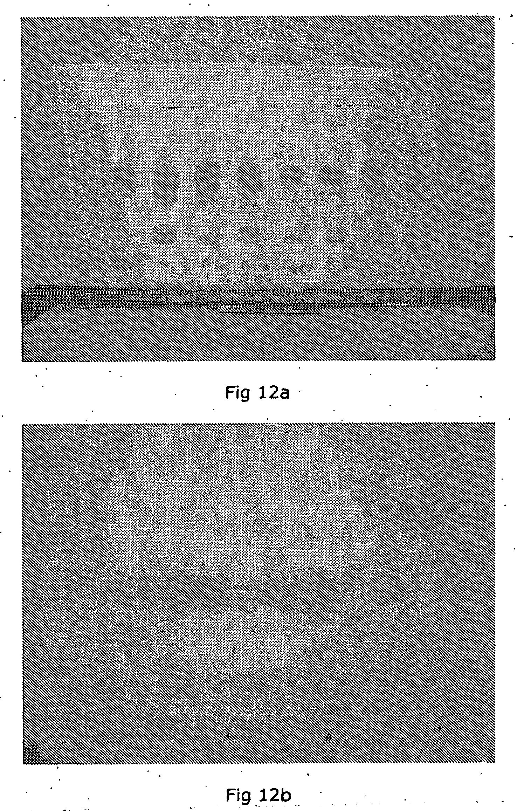

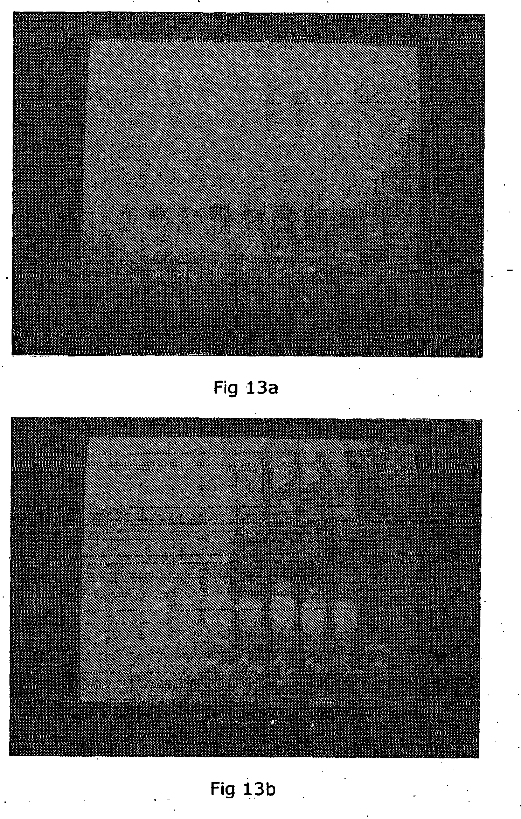

TLC 1): Plate: Cellulose; Solvent: S1; Aq & Et, T = luteoline. The results can be seen in Fig 8.
   1a: UV 366 nm Detection is seen in Fig 8a.
   1b: UV 366 nm detection after derivatisation with A is seen in Fig. 8b.
TLC 2): Plate: Cellulose; Solvent: S2. The results can be seen in Fig 9.
   2a: UV 366 nm detection (Aq & Et; T = luteoline) before derivatisation is seen in Fig. 9a.
   2b): UV 366 nm detection (Aq & Et; T = luteoline) after treatment with A is seen in Fig. 9b.
   2c: UV 366 nm detection Aq & T= apigenine before derivatisation is seen in Fig 9c.
   2d: UV 366 nm detection Aq & T= apigenine after derivatisation with F is seen in Fig 9d.
TLC 3) Plate: Cellulose; Solvent S3. Aq & Et. The results can be seen in Fig 10.
   3a: Detection: UV 366 nm before derivatisation is seen in Fig 10a.
   3b: Detection: Visual detection after derivatisation with B is seen in Fig 10b.
TLC 4) Plate: Silica; Solvent: S4; Aq & Et. The results can be seen in Fig 11.
   4a: Detection: UV 366 nm before derivatisation is seen in Fig 11a.
   4b: Detection: UV 366nm detection after derivatisation with C and 5 min heat is seen in Fig 11b.
   4c:Detection: Visual detection after derivatisation with C and 15 min heat is seen in Fig 11 c.
   4d:Detection: Visual detection after derivatisation with C and 30 min heat is seen in Fig 11d.
   4e:Detection: UV 366 nm after derivatisation with C and 30 min heat is seen in Fig 11e.
TLC 5) Plate: Silica; Solvent S5; Aq. T = glucose & rhamnose (blue). The results can be seen in Fig 12.
   5a: Detection: Visual detection after derivatisation with E is seen in Fig 12a.
   5b: Detection: Visual detection after derivatisation with D is seen in Fig 12b.
TLC 6) Plate: Cellulose; Solvent S6; Aq. T = apigenine. The results can be seen in Fig 13.
   6a: Detection: UV 366 nm before derivatisation is seen in Fig 13a.
   6b: UV 366 nm after derivatisation with F is seen in Fig 13b.

### Evaluation

### TLC 1:

1a) All the water extracts are different from the ethanolic extracts. The compound at the base-line for 3 (the sensitive 1 N exposed water extract) is more intense than for the other extracts. Chlorophyl is detected only in the ethanolic extracts placed at the base-line. Other compounds may be covered under these compounds. The dark blue may be flavonoids, detected as yellow spots in 1 b.
1b) After derivatisation of the plate 1a, the flavonoids (low Rf-values) are yellow and the simple phenolic compounds light blue (medium Rf-values). Extract 7 (R₁₁Aq) has a different composition than the other aquatic extracts of phenolic compounds. The water extract of the sensitive exposed plant do have a compound at the baseline which do not react with the reagent. This compound(s) is/are different from the other water extracts.

Flavonoids are present in the resistant plant extracts (exposed and unexposed) compared with the sensitive plant extracts. Other compounds as e.g. luteolin may be covered under these compounds. For the ethanolic extracts of 10, 12, 14 and 16 an orange spot (compound) is detected close at the base-line. This may be a flavonid (sulphated or glycoside) or chlorophyll different from the other chlorophyll.

### TLC 2:

2a): As above mentioned, the differences in the composition of the extracts water/ethanolic is not very visible. However, only chlorophyll are detected in the solvent front for the all the ethanolic extracts.
2b): After derivatisation, the flavonoids are yellow and the simple phenolic compounds light blue. The aquatic extracts of the resistant plants have a higher content of flavonoids, than the sensitive plants. Even though the Rf-value of the flavonoid in the aquatic extracts is close to luteolin, it is proved in TLC 1 1 b, that the Rf-values of the flavonoids are far from luteolin (Rf = 0).
2c & d): Only the water extracts are presented at the plates 2c and 2d. Apigenin was applicated as reference-compound. This compound is not present in the extracts. Differences in the composition of the compounds are seen in the different extracts.

### TLC 3:

3a) Both water and ethanolic extracts are present at the plates 3a & b. In 3a the differences are present between the sensitive unexposed and exposed plants as well as between the sensitive and resistant plants for both water and ethanolic extracts.
3b) In the system the amino acids are detected using ninhydrin. The amino acids are mainly dissolved in water and therefore the water extracts are the best. A strong reaction for the sensitive plants exposed to herbicide is seen and the same reaction seems to be detected for the resistant plants exposed to 10N. The sensitive unexposed seems to have the same content as the resistant plants.

### TLC 4:

4a) Concerning the UV detectable spots, the main differences were detected for the sensitive exposed water plant extract compared with the resistant plant extracts.
4b) In the extracts of R₁₀, R₁₁ and R₁₀ a spot at Rf = 0.48 was detected. This or these compounds were both water and ethanolic soluble and it was not detected for the other extracts. Since anisaldehyde detects a range of different compounds it is not possible to identify the type of compounds.
4c, d & e) In the visual picture of the plate in c & d, a special red spot was detected in the exposed sensitive plant extract and for extract of R₂₁₀ 10N exposed resistant plant extract. This kind of reaction with the same reagent was earlier detected for the plant specie *Anagalis arvensis* and it seem to be an important biomarker. Again the same composition is seen for the sensitive and the 10N exposed plants.

The UV detection of 4d in 4e confirm the red spot, however in this case it is orange fluorescence, but very visible in UV-light.

### TLC 5:

5a & b) Carbohydrate compounds are detected in these two systems. No glucose and rhamnose are detected (blue compounds in 5a). This was only seen in 5a, since the colour of the different sugars are different compared with 5b, where all the compounds are orange. A difference is detected in the sensitive exposed plants compared with the resistant plants.

### TLC 6:

6a &b) In 6a and b a different composition is detected for the sensitive exposed plant and R₁₁. Since apigenin present a dark spot in UV light before derivatisation, it is not apigenin detected after derivatisation in the plant extracts even though the Rf-values are 0 for the compounds. No general pattern for biomarkers is detected in these systems.

### Conclusion

The study confirmed that there is a difference in the composition of compounds in extracts from sensitive and resistant plant unexposed or exposed to herbicides.

### The content of phenolic compounds as simple phenolic compounds and flavonoids are interesting for further development of the project, since a new flavonoid in the sensitive plant exposed to iodosutfuron is present. This compound is not present in the biotypes of the target resistant plants only at very high exposure concentrations (10N).

The composition of amino acids is also important for the identification of target resistant unexposed and exposed plants. The same is found for the anisaldehyde derivatives, where a red spot was detected for the sensitive exposed plant (as for the highly exposed plants 10N).

### Example 5: An example of Pantone^{®}-colours

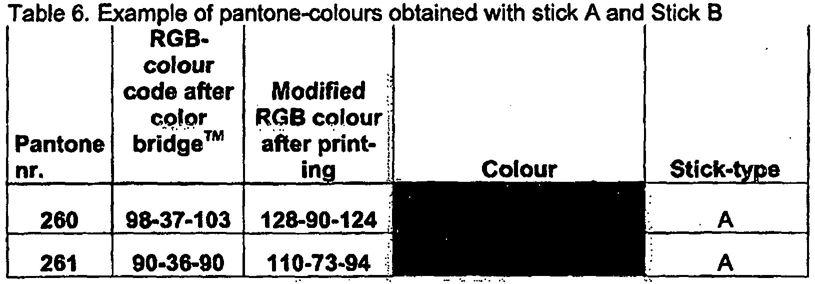

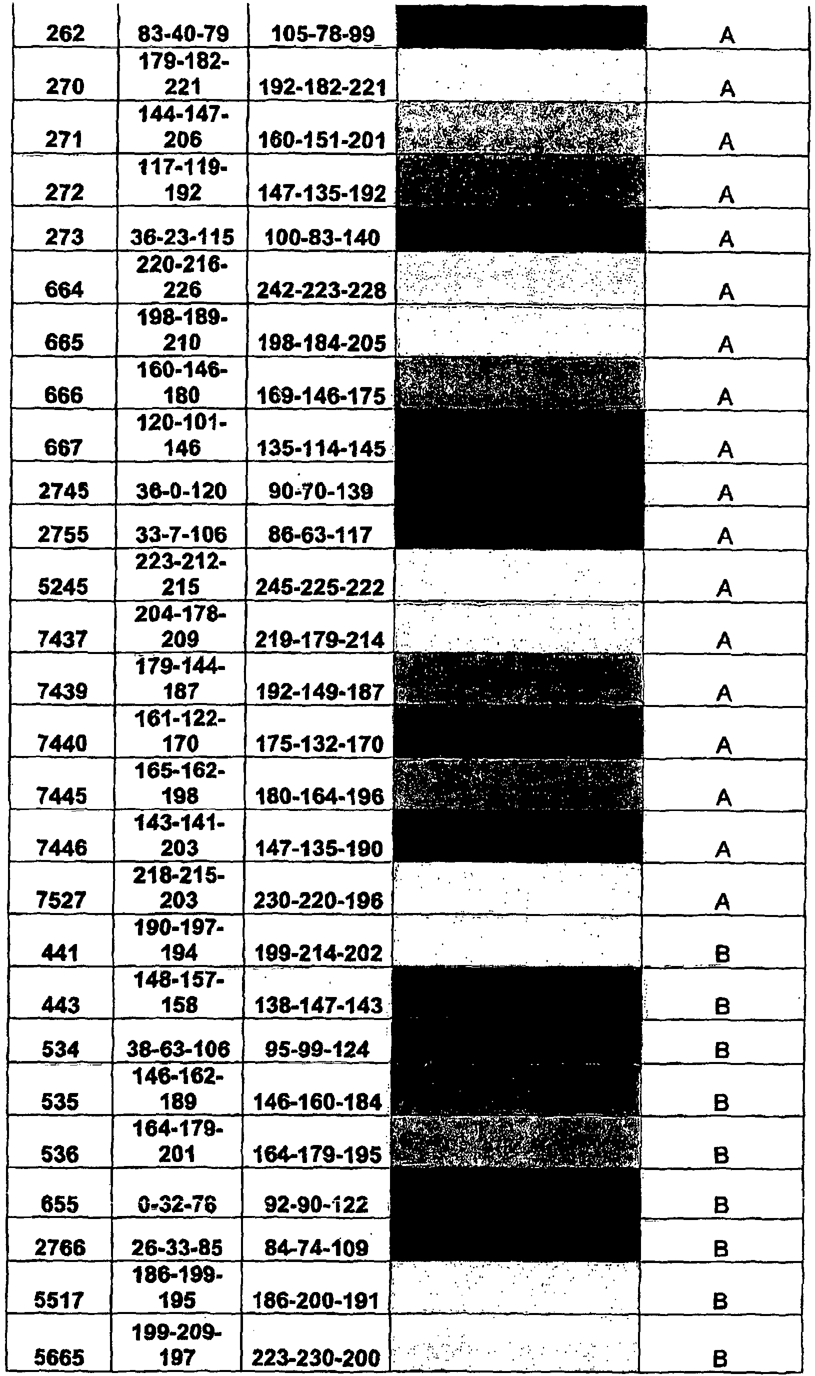

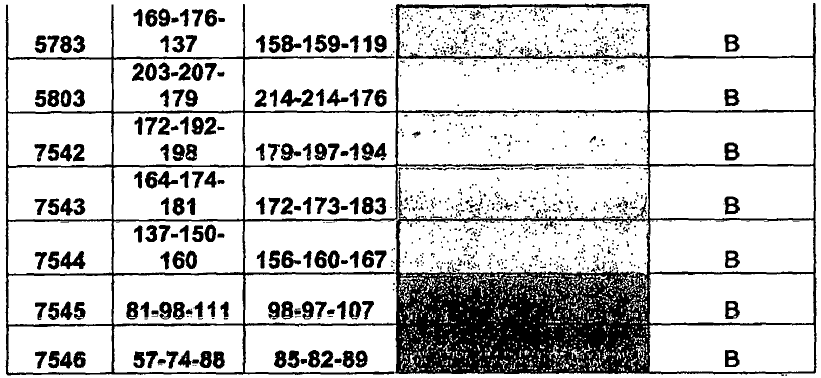

This table is only given as an example. Not all the numbers mentioned in the following tables are presented.

### Example 6: Overview of disk/stick screening program

**Table 7. Reactive groups of plants and reagents useable to obtain a colour reaction.**

| **Reagent code** | **Reactive groups** | **Recepy** | **Drops of reagent to the test** | **Treatment** | | **Extrakt conc.** | |
|---|---|---|---|---|---|---|---|
| | | | | **Heat cap¹** | **conc. sulphuric acld²** | **50 mg/ml** | **25 mg/ml** |
| **Ni** | Organic acid | 0.5% Bromocresolgreen in 96% EtOH **yellow to blue (pH = 3.8-5.4)** | **1 to 10** | - | - | | |
| **CE₁** | Organic acid | 0.5% Chlorofenolred in 96% EtOH **Yellow - violet (pH =4.8-6.7)** | **1 to 10** | - | - | | |
| **CG₁** | Organic acid | 0.5% Bromophenol blue in 96 % EtOH **Green yellow - blue violet (pH =3.0 - 4.6)** | **1 to 10** | - | - | | |
| **CH₁** | Organic acid | 0.5% Methylred in 96% EtOH **Red - yellow (pH = 4.4-6.0)** | **1 to 10** | - | - | | |
| **CI₁** | Organic acid | 0.5% Ethylred in 96% EtOH **Red - yellow** | **1 to 10** | - | - | | |
| **CF₁** | Organic acid /Lipoids | 0.5% Bromothymol blue in 96% EtOH **Yellow - blue (pH = 5.8-7.6)** | **1 to 10** | - | - | | |
| **DE₁** | Organic acid /reducing compounds | 0.5% 2.6-dichlorophenolindophenole sodium salt (Tolmanns-reagent) in 96% EtOH | **1 to 10** | - | X | | |
| **DP₁** | Organic acid /phenolic compounds | 0.5% Bromocresolpurpur in 50% EtOH **Yellow - red (pH =5.2 - 6.8)** | **1 to 10** | - | - | | |
| **DF₁** | Organic acid /phenolicco mpounds | 0.5% Bromoresolpurpur in 50% EtOH **(pH=10 with 0.1 mol/l NaOH)** | **1 to 10** | - | - | | |
| **A₁** | General compounds | 5% Vanilline in 96% EtOH | **2 to 10** | - | X | | |
| **I₁** | General compounds | 5% o-anisaldehyde in 96% EtOH | **2 to 10** | - | X | | |
| **C₁** | Amino acids | 8% Ninhydrine in 96% EtOH | **5 to 5** | X | - | | |
| **AK₁** | Amino acids | 2% Vanillin + 1% potassium hydroxide in 96% EtOH | **5 to 5** | X | - | | |
| **CC₁** | Aromatic amino acids | 10% Glucose in 50% Water and EtOH | **3 to 10** | - | X | | |
| **DL₁** | Amino acids (glyphosate) | 5% 4-chloro-7-nitrobenzofurazan in 96% EtOH | **2 to 10** | - | X | | |
| **DM₁** | Amino acids (glyphosate) | 5% 2.4-dinitrophenylhydrazine in 96% EtOH | **2 to 10** | - | X | | |
| **DN₁** | Amino acids (glyphosate) | 5%9-fluorenylmethylchloroformate in 96% EtOH | **2 to 10** | - | X | | |
| **DO₁** | Amino acids (glyphosate) | 5% Tetrabutylammoniumhydroxide in 96% EtOH | **2 to 10** | - | X | | |
| **BC₁** | N-compounds | 0.2 g iode + 0.4 g potassium iodine in 100 ml water | **5 to 5** | - | - | | |
| **E₁** | N-compounds | Solvent A: 0.85 g bismuth (III) nitrate in 10 ml acetic acid + 40 ml water. Solvent B: 8 g potassium iodine in 20 ml water. Equal parts of A and B. | **5 to 10** | - | - | | |
| **CD₁** | N-compounds, Alkaloids | 1% Ammonium ferri(III)sulfate in water | **3 to 10** | - | X | | |
| **DC₁** | Amines | 0.5% 2-Methoxy-2.4-diphenyl-3(2H)furanon (MDPF) in 96% EtOH | **2 to 10** | - | X | | |
| **DI₁** | N-compounds | 8% Potassium iodine in water | **2 to 10** | - | X | | |
| **B₁** | Phenolic compounds | 2% 2-aminoethyl-diphenylborinate in 96% EtOH | **5 to 5** | - | - | | |
| **V₁** | Phenolic compounds | 5% Ferri(III)chloride (5g in 96% ethanol + 1 ml conc. HCl) | **5 to 5** | - | - | | |
| **D₁** | Flavonoids | 5% Aluminium chloride in 96% EtOH (UV-light) | **5 to 5** | - | - | | |
| **DA₁** | Fytosterols | 0.5% Berberine chloride dihydrate in 96% EtOH | **5 to 5** | - | - | | |
| **DB₁** | Fytosterols/alkaloids | 0.5% 1.2-Naphthochinon-4-sulfonsodium salt in 96% EtOH | **2 to 10** | - | X | | |
| **DG₁** | Ketoses/glucolip ids | 300 mg Anthrone in10 ml acetic acid conc. added 20 ml 96% EtOH | **2 to10** | - | X | | |
| **DD₁** | Cationes/diazepi nes | 0.5% 8-Hydroxychinolin in 96% EtOH | **2 to 10** | - | X | | |
| **DJ,** | Carboxyl compounds/alip hatic aldehydes (C-8), glycoaldehydes, glyoxyl acid, 2,3-pentadion | 5% 2Aminodiphenyl(biphenyl-2-amine) in 96% EtOH | **2 to10** | - | X | | |
| **AD_{α1}** | Carbohydrates and glycosides | 5% α-naphthol in 96% EtOH | **2 to 10** | - | X | | |
| **AD_{β1}** | Carbohydrates and glycosides | 5% β-naphthol in 96% EtOH | **2 to 10** | - | X | | |
| **AA₁** | Carbohydrates and glycosides | 5% Naphthoresorcinol in 96% EtOH | **2 to 10** | - | X | | |
| **AE₁** | Carbohydrates and glycosides | 5% Orcinol in 96% EtOH | **2 to 10** | - | X | | |
| **AI₁** | Carbohydrates and glycosides | 5% Thymol in 96% EtOH | **2 to 10** | - | X | | |
| **U₁** | Carbohydrates and glycosides | 5% Urea in 96% EtOH | **2 to 10** | - | X | | |
| **CK₁** | Carbohydrates and glycosides | 5% 4-hydroxybenzoic acid in 96% EtOH | **2 to 10** | - | X | | |
| **DK₁** | Carbohydrates (mono-, di-) og Uronic acid | 5% 4-Aminobenzoicacid in 96% EtOH | **2 to 10** | - | X | | |
| **F₁** | Lipids | 10% Molybdatophosphoric acid in 96% EtOH | **2 to 10** | - | X | | |
| **AV₁** | Lipids | 0.5% 2'.7'-dichlorafluoresceine in 96% EtOH | **1 to 10** | - | - | | |
| **AY₁** | Lipids | 0.5% Rhodamine 6G in 96% EtOH | **1 to 10** | - | - | | |
| **DH₁** | Lipids/phosp holipids/ Steroids | 1% O-Anilinonaphthaline-1-sulfonic acid-ammonium salt in water | **2 to 10** | - | X | | |
| **AA₁** | S-compounds | 0.5% Methylene blue in 96% EtOH | **1 to 10** | - | - | | |
| **AF₁** | Reducing compounds | 6% potassium permanganate in water | **1 to 10** | - | - | | |

### 1) Test with heat cap:

In sample glas no. 1: extract + reagent (need for heat to react). The glasses were held slantingly until the solution was cooled (Approx. 10-15 min.); In samle glas no. 2: 0.50 ml water + 15 drops (glas pipette) or 24 drops (little Apodan-plastic flask) conc. sulphuric acid and heat was developed Approx. 70°C. Sample glas no. 1 was placed immediately after glas no. 2 was prepared and the glasses were held slantingly until the solution was cooled (Approx. 10-15 min.).

### 2) Reaction with heat (conc. sulphuric acid):

To the sample (containing 0.5 ml totally with reagen) 15 drops (glas pipette) or 24 drops (little Apodan-plastic flask) conc. sulphuric acid was added and heat was developed approx. 70°C.

No indication of a treatment indicates that no further treatment was necessary to obtain a reaction.

### Plant test preparation to screening methods

**Fresh:** 1500 mg fresh plant material was crushed with 30.00 ml fresh water in a little mortar. The extract was filtrered through a filter (possible with cotton in a funnel), and then through a Whatman 0.45 µm GMF w/GMF filter. The test was performed within ½-1 hour. The complete screening took approx. 2 - 2½ hour.

### Freeze-dried:

**To stick/disk:** 1500 mg freeze-dried plant material was crushed and extracted with 30.00 ml 10% ethanol in 90% water 2 hours in ultra sonic bath with ice. The extract was filtered through a Whatman 0.45 µm GMF w/GMF. The test was performed within ½-1 hour.

### Use Stick, when area/hight of area and disk when the colour only is evaluated with PANTONE^{®}- colour scale.

**Stick:** Adventec 526 filter (1x4 cm, where 1x1 cm is free and 3x1 cm is dipped into parafine). It was used when area/hight of peak with CAMAG. The stick was dipped and the PANTONE^{®}-colour was evaluated. Sticks' were placed In a holder with a hole and photographed with CAMAG (in visual light). Disk: Adventec 590 filter (d=2.5 cm) was used when only the PANTONE^{®}-colour was to be evaluated.

### Experiment 7. An example of a screening of Papaver rhoeas exposed to Hussar OD

**The numbers in the table is referring to the PANTONE^{®}-numbers. For the plant extracts 25 or 50 mg fresh frozen plant material per ml pure water is used.**

### 1) Test with heat cap:

In sample glas no. 1: extract + reagent (need for heat to react). The glasses are held slantingly until the test is cooled (Approx. 10-15 min.); In samle glas no. 2: 0.50 ml water + 15 drops (glas pipette) or 24 drops (little Apodan-plastic flask) conc. sulphuric acid and heat is developed Approx. 70°C. Pay attention it might squirt! sample glas no. 1 is placed immediately after glas no. 2 is prepared and the glasses are held slantingly until the test is cooled (Approx. 10-15 min.).

### 2) Reaction with heat (conc. sulphuric acid):

To the sample (containing 0.5 ml totally with reagen) 15 drops (glas pipette) or 24 drops (little Apodan-plastic flask) conc. sulphuric acid and heat is developed approx. 70°C.

**Table 8. Papaver rhoeas exposed to Hussar OD. The Reagents are futher described in Table 7. The plants are identified as "4", "5", "6" and "12". The results indicated for the plants are the Pantone-colours obtained with the method described below.**

| **Reagent** | **Drops of reagent to drops of the sample** | **Treatment Conc.** | | **Extract conc.** | | **4 (ID 150)** | | **5 (ID 397)** | | | **6 (ID 248)** | | | **12 (ID 406)** | | |
|---|---|---|---|---|---|---|---|---|---|---|---|---|---|---|---|---|
| | | **Heat** | **sulphuric** | | | **sensitive** | | **Target resistent** | | | **Target resistent** | | | **Target resistent** | | |
| **code** | | **cap¹** | **Acid²** | **50 mg/m l** | **25 mg/m l** | **control** | **1N** | **control** | **1 N** | **10 N** | **control** | **1 N** | **10N** | **control** | **1N** | **10 N** |
| **N₁** | **1 to 10** | - | - | x | | 3025 | 3025 | 3025 | 3025 | 3025 | 3025 | 3025 | 3025 | 3025 | 3025 | 3025 |
| **CE₁** | **1 to 10** | - | - | x | | 7508 | 7508 | 722 | 722 | 722 | 722 | 722 | 722 | 730 | 730 | 730 |
| **CG₁** | **1 to 10** | | - | x | | 2725 | 2725 | violet M | Violet M | Violet M | violet M | Violet M | Violet M | violet M | Violet M | Violet M |
| **CH₁** | **1 to 10** | - | - | x | | 472/ 714 | 714 | 7410 | 472 | 7410 | 7410 | 7410 | 486 | 714 | 714 | 7410 |
| **Cl₁** | **1 to 10** | - | - | | x | 170 | 180 | 701 | 701 | 701 | 701 | 701 | 701 | 701 | 701 | 701 |
| **CF₁** | **1 to 10** | - | - | x | | 458 | 458 | 457 | 457 | 457 | 7403 | 7403 | 7403 | 7403 | 7403 | 7403 |
| **DE₁** | **1 to 10** | - | **X** | | x | 155 | 5025 | 727 | 155 | 5035 | 726 | 726 | 5035 light | 726 | 5035 light | 5035 light |
| **DP₁** | **1 to 10** | - | - | x | | 145 | 145 | 145 | 145 | 145 | 145 | 145 | 145 | 145 | 145 | 145 |
| **DF₁** | **1 to 10** | - | - | x | | 521 | 521 | 668 | 668 | 668 | 668 | 668 | 668 | 668 | 668 | 668 |
| **A₁** | **2 to** 10 | - | **X** | x | | 7514 | 489 light | 7514 | 7514 | 4755 | 7514 | 7514 | 4755 | 7514 | 7513 | 4755 |
| **I₁** | **2 to 10** | - | **X** | x | | 7507 | 7501 | 7502 | 7501 | 7501 | 7501 | 7501 | 7500 | 7501 | 7501 | 7500 |
| **C₁** | **1 to 5** | **X** | - | x | | Clear | Clear | Clear | Clear | Clear | Clear | Clear | Clear | Clear | Clear | Clear |
| **AK₁** | **1 to 5** | **X** | - | x | | 155 | 7506 | 615 | 615 | 615 | 614 | 615 | 614 | 614 | 614 | 614 |
| **CC₁** | **3 to 10** | - | **X** | x | | 7508 | 7508 | 7502 | 7501 | 7501 light | 7502 | 7502 | 7500 | 7501 | 7500 | 7501 |
| **DL₁** | **2 to 10** | **-** | **X** | x | | 7508 | 7508 | 7502 | 7501 | 7501 light | 7502 | 7502 | 7500 | 7501 | 7500 | 7501 |
| **DM₁** | **2 to 10** | **-** | **X** | x | | 7508 | 7534 | 7502 | 7502 | 7501 light | 7501 | 7501 | 7500 | 7501 | 7501 | 7500 light |
| **DN₁** | **2 to 10** | **-** | **X** | x | | 7506 | 7534 | 7501 | 7501 | warm gray M1 | 7500 | 7501 | 7500 light | 7500 | 7500 light | 7500 light |
| **DO₁** | **2 to 10** | **-** | **X** | x | | 7502 | 7534 | 7501 | 7501 | 7534 | 7501 | 7501 | 7534 | 7501 | 7502 | 7500 light |
| **BC₁** | **1 to 5** | **-** | **-** | x | | 607 | Clear | 7500 | 7500 | 7500 | 7500 | 7500 | 7500 | 7500 | 7500 | 7500 |
| **E₁** | **5 to 10** | **-** | **-** | x | | 168 | 168 | 168 | 168 | 168 | 168 | 168 | 168 | 168 | 168 | 168 |
| **CD₁** | **3 to 10** | **-** | **X** | x | | Clear | Clear | light/ Clear | light/ clear | Clear | Clear | Clear | Clear | Clear | Clear | Clear |
| **DC₁** | **2 to 10** | **-** | **X** | x | x | 7501 | 7534 | 7502 | 7501 | 7501 light | 7502 | 7501 | 7501 light | 7501 | 7501 | 7500 light |
| **DI₁** | **2 to 10** | **-** | **X** | x | | Clear | Clear | Clear | Clears | Clear | Clear | Clear | Clear | Clear | Clear | Clear |
| **B₁** | **1 to 5** | **-** | **-** | x | | 7500 | Clear | 7500 | 7500 | light/ clear | 7500 light | 7500 | Clear | 7500 light | 7500 | Clear |
| **V₁** | **1 to 5** | **-** | **-** | x | | 609 | 609 | 617 | 617 | 617 | 617 | 617 | 617 | 617 | 617 | 617 |
| **D₁** | **1 to 5** | **-** | **-** | x | | 7500 | Clear | 7500 light | 7500 light | light/ Clear | 7500 light | 7500 light | Clear | 7500 light | 7500 light | Clear |
| **DA₁** | **1 to 5** | **-** | **-** | x | | 395 | 395 | 395 | 395 | 395 | 3955 | 3955 | 3955 | 3955 | 3955 | 3955 |
| **DB₁** | **2 to 10** | **-** | **X** | x | | 7502 | 482 | 7501 | 7501 | 7501 light | 7501 | 7501 | 7500 | 7501 | 7501 | 7500 |
| **DG₁** | **2 to 10** | - | **X** | x | | 7501 | 482 | 7501 | 7501 | 7501 | 7501 | 7501 | 7501 light | 7501 | 7501 | 7501 light |
| **DD₁** | **2 to 10** | - | **X** | x | | 7501 | 482 | 7502 | 7502 | 7501 | 7501 | 7501 | 7500 light | 7501 | 7501 | 7500 light |
| **DJ₁** | **2 to 10** | - | **X** | x | | 7501 | 4755 | 7502 | 7502 | 7501 | 7502 | 7502 | 7501 | 4685 | 4685 | 4685 light |
| **AD_{α1}** | **2 to 10** | - | **X** | x | | 5275 | 5783 | 7545 | 7545 | 5773 | 535 | 535 | 5783 | 5545 | 5625 | 5767 |
| **AD_{β1}** | **2 to 10** | - | **X** | x | | 7504 | 7502 | 7504 | 17504 | 7503 | 7502 | 7502 | 7503 | 7503 | 4525 | 4525 |
| | | | | | | | | light | light | | dark | dark | | | | |
| **AA₁** | **2 to 10** | **-** | **X** | | x | 505 | 4985 light | 505 | 505 | 4985 | 4985 | 505 | 4985 greyish | 4985 | 4985 qreyish | 4985 greyish |
| **AE₁** | **2 to 10** | **-** | **X** | | x | 7502 | 5875 | 7502 | 7502 | 7503 light | 7502 | 7502 | 7503 light | 7501 | 7501 | 7501 |
| **AI₁** | **2 to 10** | **-** | **X** | x | | 486 | 4685 | 7514/ 486 | 727 | 7528 | 726 | 727 | 726 | 5005 | 727 | 726 |
| **U₁** | **2 to 10** | **-** | **X** | x | | 7502 | 4755 | 7502 | 7501 | 4755 | 7501 | 7501 | 7500 | 7501 | 7501 | 7500 |
| **CK₁** | **2 to 10** | **-** | **X** | x | | 7502 | 4755 | 7502 | 7502 | 7534 light | 7501 | 7501 | 7501 light | 7501 | 7501 light | light/ clear |
| **DK₁** | **2 to 10** | **-** | **X** | x | | 7502 | 4755 | 7502 | 7502 | 7534 light | 7502 | 7502 | 7501 light | 726 | 726 | 7501 light |
| **F₁** | **2 to 10** | **-** | **x** | x | | 7501 | warm grey M1 | 7501 | 7500 | 7534 | 7500 | 7500 | 7500 | 7500 | 7500 | 7500 |
| **AV₁** | **1 to 10** | **-** | **-** | x | | 116 | 116 | 108 | 108 | 108 | 108 | 108 | 108 | 108 | 108 | 108 |
| **AY₁** | **1 to 10** | **-** | **-** | x | | 178 | 178 | 178 | 178 | 178 | 178 | 178 | 178 | 178 | 178 | 178 |
| **DH₁** | **2 to 10** | **-** | **X** | x | | 4735 | 7501 | 7530 | 7529 | 7502 | 7530 | 7530 | 7527 | 7530 | 7527 | 7527 |
| **AA₁** | **1 to 10** | **-** | **-** | x | | 3015 | 3015 | 3015 | 3015 | 3015 | 3015 | 3015 | 3015 | 3015 | 3015 | 3015 |
| **AF₁** | **1 to 10** | **-** | **-** | x | | 512 | 512 | 512 | 512 | 512 | 512 | 512 | 512 | 512 | 512 | 512 |

### 1) Test with heat cap:

In sample glass no. 1: extract + reagent (need for heat to react). The glasses were held slantingly until the solution was cooled (Approx. 10-15 min.); In samle glas no. 2: 0.50 ml water + 15 drops (glas pipette) or 24 drops (little Apodan-plastic flask) conc. sulphuric acid and heat was developed Approx. 70°C. Sample glas no. 1 was placed immediately after glass no. 2 was prepared and the glasses were held slantingly until the solution was cooled (Approx. 10-15 min.).

### 2) Reaction with heat (conc. sulphuric acid):

To the sample (containing 0.5 ml totally with reagent) 15 drops (glas pipette) or 24 drops (little Apodan-plastic flask) conc. sulphuric acid was added and heat was developed approx. 70°C.

No indication of a treatment indicates that no further treatment was necessary to obtain a reaction.

## Claims

1. A method of testing for a specific herbicide resistance in a specific living plant, said method comprising:
- providing an extract of said specific living plant, said extract comprising at least one phytochemical compound,
- detecting the at least one phytochemical compound by visually observing said at least one phytochemical compound on a solid support, or visualising them by visual and/or UV-light,
- correlating said visualising of said at least one phytochemical compound to a standard visualising scale of said at least one phytochemical compound from a resistant plant, which is of the same biotype as said specific living plant, and
- hereby assessing whether said specific living plant has developed resistance towards said specific herbicide.

2. The method according to claim 1, wherein the method prior to the step of providing an extract of said part of a specific living plant further comprises
- exposing said at least one specific living plant to a specific herbicide,
- after a period of time selecting at least a part of the specific living plant which is not dead due to the exposure to said specific herbicide,

3. The method according to claim 1 or 2, wherein the method prior to visualising the at least one group of phytochemical compounds further comprises,
- providing at least one chemical reagent applicable for a chemical reaction with said at least one group of phytochemical compounds, and
- provoking a chemical reaction between said chemical reagents and phytochemical compounds of said specific living plant material, and
where said visualising of at least one group of phytochemical compounds is a detection of a result of said chemical reaction.

4. The method according to claim 3, wherein the chemical reagent is based on one or more of the compounds chlorofenolred, methylred, ethylred, bromothymol blue, 2.6-dichlorophenolindophenole sodium salt, bromocresolpurpur, ninhydrine, vanillin + potassium hydroxide, glucose, 4-chloro-7-nitrobenzofurazan, 2.4-dinitrophenylhydrazine, 9-fluorenylmethylchloroformate, tetrabutylammoniumhydroxide, iode + potassium iodine, bismuth (III) nitrate, Ammonium ferri(III)sulphate, 2-methoxy-2.4-diphenyl-3(2H)furanon (MDPF), 2-aminoethyl-diphenylborinate, ferri(III)chloride, aluminium chloride, berberine chloride dihydrate, 1.2-naphthochinon-4-sulfonsodium salt, anthrone, 8-hydroxychinolin, 2-aminodiphenyl(biphenyl-2-amine), orcinol, urea, 4-hydroxybenzoic acid, 4-aminobenzoic acid, molybdatophosphoric acid, 2'.7'-dichlorofluoresceine, 8-anilinonaphthaline-1-sulfonic acid-ammonium salt, rhodamine, iod, potassium iodide, ammoniummolybdattin(II) chloride, cobalt(II) chloride, palladium(II) chloride, nitrate potassium iodide, vanillin, sulphuric acid, naphtoresorcinol, methylene blue, β-naphtol, thymol, fluorescein, ammonia, bromocresol green, bromophenol blue, potassium permanganate, 2,7-dichlorofluorescein, Rodamin 6G, Diphenyl boric acid 2-aminoethylester, phosphoric acid, iod, potassium iodide, ammonium-molybdattin(II) chloride, cobalt(II) chloride, palladium(II) chloride, ninhydrin, 1-naphthol, bismuth(III) nitrate potassium iodide, molybdat phosphor acid, rodamin B, anise aldehyde, silver nitrate, ferro(III) chloride, zinkchloride and chemicals or mixtures hereof.

5. The method according to any of the preceding claims, wherein the assessment of the method is selected from the group of qualitative and/or quantitative and/or semi-quantitative assessment or a combination thereof.

6. The method according to any of the preceding claims, wherein the use of chemical reagents and solid support comprises the following steps:
- contacting an extract of said specific living plant with at least one chemical reagent,
- providing a chemical reaction between the extract of material from the living plant and the at least one chemical reagent,
- contacting a solid support with the material from the specific living plant chemically reacted with the at least one chemical reagent, hereby
- obtaining a solid support with a colour (visual or detection in UV-light)
- comparing the colour and colour intensity with a standard visualising scale,
- evaluating existence of resistance in said specific living plant.

7. The method according to any of the preceding claims, wherein the testing is performed by separating individual compounds belonging to said at least one group of phytochemical compounds obtained from the living plant.

8. The method according to any of the preceding claims, wherein the testing is performed on at least one non-separated group of phytochemical compounds obtained from the specific living plant.

9. The method according to any one of the preceding claims, wherein the detection is performed by an analytical method selected from the group consisting of High Performance Liquid Chromatography (HPLC), Gas Chromatography (GC) or Mass Spectrometry (MS) or a or a combination of said analytical methods.

10. A method of providing a standard visualising scale for material from a specific living plant that has or has not been exposed to a specific herbicide, said method comprises the steps of:
- subjecting at least one specific living plant to known types and known amounts of a specific herbicide or to no herbicide,
- obtaining material from said living plant,
- determining the phytochemical responses of said material from said specific living plant for each specific herbicide type and/or for each amount of herbicide,
and
- obtaining at least one standard result relating to said specific herbicide type and/or to said amount of specific herbicide.

11. The method according to claim 10, wherein said phytochemical response is based on phytochemical compounds in the living plant which are correlated with occurrence of resistance in said living plant.

12. The method according to any of claim 10 to 11, further including the features of claims 1 to 9.

13. An assay kit for testing for herbicide resistance in a living plant, said assay kit comprising:
- at least one filter paper,
- at least one solvent,
- at least one squeezing means,
- at least one standard visualising scale of at least one phytochemical compound of a specific living plant, which has been exposed to a specific herbicide and/or known amounts of said specific herbicide, for determining whether a plant has developed herbicide resistance,
- at least one container.

14. The assay kit according to claim 13, further comprising one or more of the components selected from the group of
- at least one chemical reagent,
- at least one mortar with pistil and/or at least one box with balls to shake and/or at least one hand-press
- at least one pipette,
- at least one UV-lamp,
- at least one heater and/or at least one warm cap made of chemical reagents and solvents,
- at least one balance,
- at least one scissor,
- at least one pair of forceps,
- at least one plastic bag,
- at least one identification information to identify plant species,
- at least one instruction describing how to use the assay kit,
- at least one syringe,
- at least one filter
- at least one Thin Layer Chromatography plate.

15. The assay kit according to claim 13 or 14, wherein said kit comprises
- 3 pieces of filter paper,
- 3 boxes with lids and each with 4 glass balls
- 6 glasses,
- 3 syringes each with 13.5 mL solvent,
- 1-3 containers with chemical reagents,
- 3 pipettes,
- 1 balance,
- 1 scissor,
- 1 pair of forceps,
- 3 plastic bags,
- 1 identification information to identify plant species,
- 1 instruction describing how to use the assay kit including a standard colour scale,
- 3 filters.

16. Use of the assay kit as defined in the claims 12-15 for testing for herbicide resistance in plants.

## Patentansprüche

1. Verfahren zum Prüfen einer spezifischen lebenden Pflanze auf eine spezifische Herbizidresistenz, wobei das Verfahren umfasst:
- Bereitstellen eines Extraktes der spezifischen lebenden Pflanze, wobei der Extrakt wenigstens eine phytochemische Verbindung umfasst,
- Detektieren der wenigstens einen phytochemischen Verbindung mittels visueller Überwachung der wenigstens einen phytochemischen Verbindung auf einem festen Träger oder mittels Visualisieren dieser mit sichtbarem und/oder UV-Licht,
- Korrelieren der Visualisierung der wenigstens einen phytochemischen Verbindung mit einer Standardvisualisierungsskala der wenigstens einen phytochemischen Verbindung einer resistenten Pflanze, die vom gleichen Biotyp ist wie die spezifische lebende Pflanze, und
- dadurch Beurteilen, ob die spezifische lebende Pflanze gegenüber dem spezifischen Herbizid eine Resistenz entwickelt hat.

2. Verfahren nach Anspruch 1, wobei das Verfahren vor dem Schritt des Bereitstellens eines Extraktes des Teils einer spezifischen lebenden Pflanze ferner umfasst:
- Aussetzen der wenigstens einen spezifischen lebenden Pflanze einem spezifischen Herbizid,
- nach einer Zeitdauer Auswählen wenigstens eines Teils der spezifischen lebenden Pflanze, der durch das Aussetzen dem spezifischen Herbizid nicht abgestorben ist.

3. Verfahren nach Anspruch 1 oder 2, wobei das Verfahren vor dem Visualisieren der wenigstens einen Gruppe von phytochemischen Verbindungen ferner umfasst:
- Bereitstellen von wenigstens einem chemischen Reagenz, das für eine chemische Reaktion mit der wenigstens einen Gruppe von phytochemischen Verbindungen anwendbar ist, und
- Provozieren einer chemischen Reaktion zwischen den chemischen Reagenzien und den phytochemischen Verbindungen des spezifischen lebenden Pflanzenmaterials, und
wobei das Visualisieren der wenigstens einen Gruppe von phytochemischen Verbindungen eine Detektion eines Ergebnisses der chemischen Reaktion ist.

4. Verfahren nach Anspruch 3, wobei das chemische Reagenz auf einem oder mehreren der folgenden Stoffe basiert: Chlorphenolrot, Methylrot, Ethylrot, Bromthymolblau, 2,6-Dichlorphenolindophenolnatriumsalz, Bromcresolpurpur, Ninhydrin, Vanillin + Kaliumhydroxid, Glucose, 4-Chlor-7-nitrobenzofurazan, 2,4-Dinitrophenylhydrazin, 9-Fluorenylmethylchlorformat, Tetrabutylammoniumhydroxid, lod + Kaliumiodin, Bismut-(III)-nitrat, Ammoniumeisen-(III)-sulfat, 2-Methoxy-2,4-Diphenyl-3(2H)-furanon (MDPF), 2-Aminoethyldiphenylborinat, Eisen-(III)-chlorid, Aluminiumchlorid, Berberinchloriddihydrat, 1,2-Naphthochinon-4-sulfonnatriumsalz, Anthron, 8-Hydroxychinolin, 2-Aminodiphenyl(biphenyl-2-amin), Orcinol, Urea, 4-Hydroxybenzoesäure, 4-Aminobenzoesäure, Molybdatphosphorsäure, 2',7'-Dichlorfluorescein, 8-Anilinnaphthalin-1-sulfonsäure-Ammoniumsalz, Rhodamin, lod, Kaliumiodid, Ammoniummolybdat-Zinn-(11)-chlorid, Kobalt-(II)-chlorid, Palladium-(II)-chlorid, Nitratekaliumiodid, Vanillin, Schwefeläsure, Naphtoresorcin, Methylenblau, β-Naphtol, Thymol, Fluorescein, Ammoniak, Bromcresolgrün, Bromphenolblau, Kaliumpermanganat, 2,7-Dichlorfluorescein, Rhodamin 6G, Diphenylborsäure-2-aminoethylester, Phosphorsäure, lod, Kaliumiodid, Ammoniummolybdat-Zinn-(II)-chlorid, Kobalt-(II)-chlorid, Palladium-(II)-chlorid, Ninhydrin, 1-Naphthol, Bismut-(III)-nitratkaliumiodid, Molybdatphosphorsäure, Rhodamin B, Anisaldehyd, Silbernitrat, Eisen-(III)-chlorid, Zinkchlorid und Chemikalien oder Mischungen davon.

5. Verfahren nach einem der vorhergehenden Ansprüche, wobei die Bewertung des Verfahrens aus der Gruppe der qualitativen und/oder quantitativen und/oder semi-quantitativen Bewertung oder einer Kombination davon ausgewählt ist.

6. Verfahren nach einem der vorhergehenden Ansprüche, wobei die Verwendung von chemischen Reagenzien und festem Trägerstoff die folgenden Schritte umfasst:
- in Kontakt bringen eines Extraktes der spezifischen lebenden Pflanze mit wenigstens einem chemischen Reagenz,
- Unterstützen einer chemischen Reaktion zwischen dem Extrakt des Materials von der lebenden Pflanze und dem wenigstens einen chemischen Reagenz,
- in Kontakt bringen eines festen Trägers mit dem Material von der spezifischen lebenden Pflanze, das mit dem wenigstens einen chemischen Reagenz chemisch reagiert hat, dadurch
- Gewinnen eines festen Trägers mit einer Farbe (sichtbar oder Detektion unter UV-Licht),
- Vergleichen der Farbe und der Farbintensität mit einer Standardvisualisierungsskala,
- Evaluieren des Vorhandenseins einer Resistenz in der spezifischen lebenden Pflanze.

7. Verfahren nach einem der vorhergehenden Ansprüche, wobei das Prüfen durch Auftrennen der einzelnen Komponenten, die zu der wenigstens einen Gruppe von aus der lebenden Pflanze gewonnenen phytochemischen Verbindungen gehören, durchgeführt wird.

8. Verfahren nach einem der vorhergehenden Ansprüche, wobei das Prüfen an wenigstens einer nicht-aufgetrennten Gruppe von aus der lebenden Pflanze gewonnenen phytochemischen Verbindungen durchgeführt wird.

9. Verfahren nach einem der vorhergehenden Ansprüche, wobei die Detektion mittels eines analytischen Verfahrens durchgeführt wird, das aus der aus Hochleistungsflüssigkeitschromatographie (HPLC), Gaschromatographie (GC) oder Massenspektrometrie (MS) oder einer Kombination der analytischen Verfahren bestehenden Gruppe ausgewählt ist.

10. Verfahren zum Bereitstellen einer Standardvisualisierungsskala für Material von einer spezifischen lebenden Pflanze, die einem spezifischen Herbizid ausgesetzt wurde oder nicht, wobei das Verfahren die Schritte umfasst:
- Aussetzen der wenigstens einen spezifischen lebenden Pflanze bekannten Typen und bekannten Mengen eines spezifischen Herbizids oder keinem Herbizid,
- Gewinnen von Material aus der spezifischen lebenden Pflanze,
- Bestimmen der phytochemischen Reaktionen des Materials aus der spezifischen lebenden Pflanze für jeden spezifischen Herbizidtyp und/oder jede Herbizidmenge, und
- Gewinnen wenigstens eines Standardergebnisses, das dem spezifischen Herbizidtyp und/oder der spezifischen Herbizidmenge entspricht.

11. Verfahren nach Anspruch 10, wobei die phytochemische Reaktion auf phytochemischen Verbindungen in der lebenden Pflanze basiert, die mit dem Auftreten einer Resistenz in der lebenden Pflanze korreliert.

12. Verfahren nach Anspruch 10 oder 11, ferner umfassend die Merkmale der Ansprüche 1 bis 9.

13. Probenkit zum Prüfen einer Herbizidresistenz einer lebenden Pflanze, wobei das Probenkit umfasst:
- wenigstens ein Filterpapier,
- wenigstens ein Lösungsmittel,
- wenigstens ein Quetschmittel,
- wenigstens eine Standardvisualisierungsskala wenigstens einer phytochemischen Verbindung einer spezifischen lebenden Pflanze, die einem spezifischen Herbizid und/oder einer bekannten Menge des Herbizids ausgesetzt wurde, um zu bestimmen, ob eine Pflanze eine Herbizidresistenz entwickelt hat,
- wenigstens einen Behälter.

14. Probenkit nach Anspruch 13, ferner umfassend eine oder mehrere Komponente/n, die ausgewählt ist/sind aus der Gruppe aus:
- wenigstens einem chemischen Reagenz,
- wenigstens einem Mörser mit Stößel und/oder wenigstens einer Box mit Kugeln zum Schütteln und/oder wenigstens einer Handpresse,
- wenigstens einer Pipette,
- wenigstens einer UV-Lampe,
- wenigstens einem Heizgerät und/oder wenigstens einem aus chemischen Reagenzien und Lösungsmitteln hergestellten Wärmedeckel,
- wenigstens einer Waage,
- wenigstens einer Schere,
- wenigstens einer Zange,
- wenigstens einer Plastiktüte,
- wenigstens einer Identifizierungsinformation, um Pflanzenarten zu identifizieren,
- wenigstens einer Anleitung, die beschreibt, wie das Probenkit anzuwenden ist,
- wenigstens einer Injektionsspritze,
- wenigstens einem Filter,
- wenigstens einer Dünnschichtchromatographieplatte.

15. Probenkit nach Anspruch 13 oder 14, wobei das Kit ferner umfasst:
- 3 Stück Filterpapier,
- 3 Boxen mit Deckel und jeweils mit 4 Glaskugeln,
- 6 Gläser,
- 3 Injektionsspritzen mit jeweils 13,5 ml Lösungsmittel,
- 1-3 Behälter mit chemischen Reagenzien,
- 3 Pipetten,
- 1 Waage,
- 1 Schere,
- 1 Zange,
- 3 Plastiktüten,
- 1 Identifizierungsinformation, um Pflanzenarten zu identifizieren,
- 1 Anleitung, die beschreibt wie das Testkit anzuwenden ist, inklusive einer Standardfarbskala,
- 3 Filter.

16. Verwendung des Probenkits nach einem der Ansprüche 12 bis 15 zum Prüfen einer Herbizidresistenz von Pflanzen.

## Revendications

1. Procédé de test d'une résistance contre un herbicide spécifique chez une plante vivante spécifique, ledit procédé comprenant :
- la fourniture d'un extrait de ladite plante vivante spécifique, ledit extrait comprenant au moins un composé phytochimique,
- la détection du au moins un composé phytochimique en observant visuellement ledit au moins un composé phytochimique sur un support solide ou en les visualisant sous une lumière visible et/ou UV,
- la corrélation de ladite visualisation dudit au moins un composé phytochimique avec une échelle normalisée de visualisation dudit au moins un composé phytochimique provenant d'une plante résistante, qui est du même biotype que ladite plante vivante spécifique, et
- le fait de déterminer de cette manière si ladite plante vivante spécifique a développé une résistance contre ledit herbicide spécifique.

2. Procédé selon la revendication 1, dans lequel le procédé, avant l'étape de fourniture d'un extrait de ladite partie d'une plante vivante spécifique, comprend en outre :
- l'exposition de ladite au moins une plante vivante spécifique à un herbicide spécifique,
- après une période de temps, la sélection d'au moins une partie de la plante vivante spécifique qui n'est pas morte à cause de l'exposition audit herbicide spécifique.

3. Procédé selon la revendication 1 ou 2, dans lequel le procédé, avant la visualisation du au moins un groupe de composés phytochimiques, comprend en outre :
- la fourniture d'au moins un réactif chimique utilisable pour une réaction chimique avec ledit au moins un groupe de composés phytochimiques, et
- la provocation d'une réaction chimique entre lesdits réactifs chimiques et les composés phytochimiques dudit matériel de la plante vivante spécifique, et
ladite visualisation d'au moins un groupe de composés phytochimiques est une détection d'un résultat de ladite réaction chimique.

4. Procédé selon la revendication 3, dans lequel le réactif chimique est basé sur un ou plusieurs des composés suivants : le rouge de chlorophénol, le rouge de méthyle, le rouge d'éthyle, le bleu de bromothymol, le sel de sodium du 2,6-dichlorophénolindophénol, le pourpre de bromocrésol, la ninhydrine, la vanilline + l'hydroxyde de potassium, le glucose, le 4-chloro-7-nitrobenzofurazane, la 2,4-dinitrophénylhydrazine, le chloroformiate de 9-fluorénylméthyle, l'hydroxyde de tétrabutylammonium, l'iode + l'iodure de potassium, le nitrate de bismuth (III), le sulfate d'ammonium et de fer (III), la 2-méthoxy-2,4-diphényl-3(2H)furanone (MDPF), le borinate de 2-aminoéthyl-diphényle, le chlorure de fer (III), le chlorure d'aluminium, le chlorure de berbérine dihydraté, le sel de sodium de la 1,2-naphtochinon-4-sulfone, l'anthrone, la 8-hydroxy-quinoléine, la 2-aminodiphényl(biphényl-2-amine), l'orcinol, l'urée, l'acide 4-hydroxybenzoïque, l'acide 4-aminobenzoïque, l'acide molybdatophosphorique, la 2',7'-dichlorofluorescéine, le 8-anilinonaphtaline-1-sulfonate d'ammonium, la rhodamine, l'iode, l'iodure de potassium, le molybdate d'ammonium, le chlorure d'étain (II), le chlorure de cobalt (II), le chlorure de palladium (II), l'iodure de nitrate de potassium, la vanilline, l'acide sulfurique, le naphtorésorcinol, le bleu de méthylène, le β-naphtol, le thymol, la fluorescéine, l'ammoniac, le vert de bromocrésol, le bleu de bromophénol, le permanganate de potassium, la 2,7-dichlorofluorescéine, la rhodamine 6G, le diphénylborate de 2-aminoéthyle, l'acide phosphorique, l'iode, l'iodure de potassium, le molybdate d'ammonium, le chlorure d'étain (II), le chlorure de cobalt (II), le chlorure de palladium (II), la ninhydrine, le 1-naphtol, le nitrate de bismuth (III), l'iodure de potassium, le molybdate/acide phosphorique, la rhodamine B, l'anisaldéhyde, le nitrate d'argent, le chlorure de fer (III), le chlorure de zinc et leurs produits chimiques ou leurs mélanges.

5. Procédé selon l'une quelconque des revendications précédentes, dans lequel l'évaluation du procédé est choisie dans le groupe comprenant l'évaluation qualitative et/ou l'évaluation quantitative et/ou l'évaluation semi-quantitative ou une combinaison de celles-ci.

6. Procédé selon l'une quelconque des revendications précédentes, dans lequel l'utilisation des réactifs chimiques et du support solide comprend les étapes suivantes :
- la mise en contact d'un extrait de ladite plante vivante spécifique avec au moins un réactif chimique,
- la fourniture d'une réaction chimique entre l'extrait de matériel provenant de la plante vivante et le au moins un réactif chimique,
- la mise en contact d'un support solide avec le matériel provenant de ladite plante vivante spécifique ayant réagi par voie chimique avec le au moins un réactif chimique, et de cette manière
- l'obtention d'un support solide présentant une couleur (visible ou détection sous lumière UV),
- la comparaison de la couleur et de l'intensité de la couleur avec une échelle normalisée de visualisation,
- la détermination de l'existence d'une résistance dans ladite plante vivante spécifique.

7. Procédé selon l'une quelconque des revendications précédentes, dans lequel le test est réalisé en séparant les composés individuels appartenant audit au moins un groupe de composés phytochimiques obtenus à partir de la plante vivante.

8. Procédé selon l'une quelconque des revendications précédentes, dans lequel le test est réalisé sur au moins un groupe non séparé de composés phytochimiques obtenus à partir de la plante vivante spécifique.

9. Procédé selon l'une quelconque des revendications précédentes, dans lequel la détection est réalisée par un procédé analytique choisi dans le groupe constitué par la chromatographie liquide haute performance (CLHP), la chromatographie en phase gazeuse (CG) et la spectrométrie de masse (SM) ou une combinaison desdits procédés analytiques.

10. Procédé de fourniture d'une échelle normalisée de visualisation pour un matériel provenant d'une plante vivante spécifique qui a été exposée ou non à un herbicide spécifique, ledit procédé comprenant les étapes suivantes :
- la soumission d'au moins une plante vivante spécifique à des types connus d'herbicide spécifique et à des quantités connues d'un herbicide spécifique ou à aucun herbicide,
- l'obtention d'un matériel provenant de ladite plante vivante,
- la détermination des réponses phytochimiques dudit matériel provenant de ladite plante vivante spécifique pour chaque type d'herbicide spécifique et/ou pour chaque quantité d'herbicide, et
- l'obtention d'au moins un résultat normalisé en rapport avec ledit type d'herbicide spécifique et/ou avec ladite quantité d'herbicide spécifique.

11. Procédé selon la revendication 10, dans lequel ladite réponse phytochimique est basée sur des composés phytochimiques dans la plante vivante qui sont corrélés avec la survenue d'une résistance dans ladite plante vivante.

12. Procédé selon l'une quelconque des revendications 10 et 11, comprenant en outre les caractéristiques des revendications 1 à 9 ;

13. Kit d'essai permettant de tester la résistance contre un herbicide dans une plante vivante, ledit kit d'essai comprenant :
- au moins un papier-filtre,
- au moins un solvant,
- au moins un moyen d'écrasement,
- au moins une échelle normalisée de visualisation d'au moins un composé phytochimique d'une plante vivante spécifique, qui a été exposée à un herbicide spécifique et/ou à des quantités connues dudit herbicide spécifique, permettant de déterminer si une plante a développé une résistance contre un herbicide,
- au moins un récipient.

14. Kit d'essai selon la revendication 13, comprenant en outre un ou plusieurs des composants choisis parmi :
- au moins un réactif chimique,
- au moins un mortier et pilon et/ou au moins une boîte à secouer contenant des billes et/ou au moins une presse à main,
- au moins une pipette,
- au moins une lampe UV,
- au moins un dispositif de chauffage et/ou au moins une coiffe chauffante faite de réactifs chimiques et de solvants,
- au moins une balance,
- au moins une paire de ciseaux,
- au moins une paire de pinces,
- au moins un sac en plastique,
- au moins une information d'identification servant à identifier une espèce végétale,
- au moins une instruction décrivant comment utiliser le kit d'essai,
- au moins une seringue,
- au moins un filtre,
- au moins une plaque de chromatographie sur couche mince.

15. Kit d'essai selon la revendication 13 ou 14, dans lequel ledit kit comprend :
- 3 morceaux de papier-filtre,
- 3 boîtes avec des bouchons, contenant chacune 4 billes de verre,
- 6 verres,
- 3 seringues, contenant chacune 13,5 ml de solvant,
- 1 à 3 récipients contenant des réactifs chimiques,
- 3 pipettes,
- 1 balance,
- 1 paire de ciseaux,
- 1 paire de pinces,
- 3 sacs en plastique,
- 1 information d'identification servant à identifier une espèce végétale,
- 1 instruction décrivant comment utiliser le kit d'essai comprenant une échelle normalisée de couleurs,
- 3 filtres.

16. Utilisation du kit d'essai tel que défini dans les revendications 12 à 15, pour tester une résistance contre un herbicide chez des plantes.
